(19) Europäisches Patentamt
European Patent Office
Office européen des brevets

(11) EP 4 129 984 A1

(12) **EUROPEAN PATENT APPLICATION**
published in accordance with Art. 153(4) EPC

(43) Date of publication:
**08.02.2023 Bulletin 2023/06**

(21) Application number: **21780685.0**

(22) Date of filing: **29.03.2021**

(51) International Patent Classification (IPC):
***C07D 233/90*** *(1974.07)* ***C08G 18/20*** *(1974.07)*
***C08G 18/76*** *(1974.07)* ***C08G 18/80*** *(1974.07)*

(52) Cooperative Patent Classification (CPC):
**C07D 233/90; C08G 18/20; C08G 18/76; C08G 18/80**

(86) International application number:
**PCT/JP2021/013184**

(87) International publication number:
**WO 2021/200783 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.03.2020 JP 2020061166**

(71) Applicant: **Koei Chemical Company, Limited**
**Sodegaura-shi, Chiba 299-0266 (JP)**

(72) Inventors:
- **MIYAGI, Motoyoshi**
  **Sodegaura-shi, Chiba 299-0266 (JP)**
- **ONODA, Mitsuki**
  **Sodegaura-shi, Chiba 299-0266 (JP)**

(74) Representative: **Müller-Boré & Partner Patentanwälte PartG mbB**
**Friedenheimer Brücke 21**
**80639 München (DE)**

# (54) AMIDATE COMPOUND, PRODUCTION METHOD THEREFOR, BLOCKING-AGENT DISSOCIATION CATALYST, AND THERMALLY CURABLE RESIN COMPOSITION

(57) The invention provides a method for producing an amidate compound, comprising reacting an imidazolium carboxylic acid salt represented by the following formula (1):

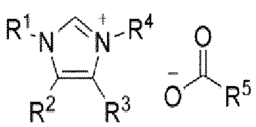 (1)

wherein $R^1$ to $R^5$ are as defined in the specification, with a polyisocyanate compound represented by the following formula (2):

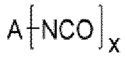 (2)

wherein A and x are as defined in the specification, and wherein the produced amidate compound is represented by the following formula (3):

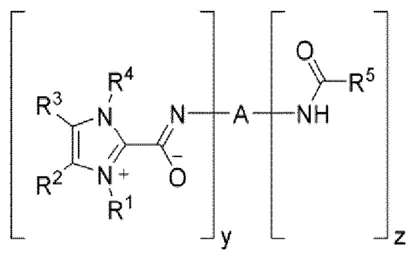 (3)

wherein y, z, A, and $R^1$ to $R^5$ are as defined in the specification.

EP 4 129 984 A1

## Description

### Technical Field

The present invention relates to an amidate compound, a production method for the compound, a blocking agent dissociation catalyst, and a thermosetting resin composition.

### Background Art

Conventionally known methods for producing an amidate compound include a method comprising reacting an N-heterocyclic carbene (hereinafter referred to as "NHC carbene") with an isocyanate (Non-patent Literature (NPL) 1).
Patent Literature (PTL) 1 discloses an amidate compound that can be used as a blocking agent dissociation catalyst.

### Citation List

#### Patent Literature

PTL 1: WO2019/065953A1

#### Non-patent Literature

NPL 1: Struct. Chem., 2013, vol. 24, pp. 2059-2068

### Summary of Invention

#### Technical Problem

The method disclosed in NPL 1, which comprises reacting an NHC carbene with an isocyanate, requires the use of an NHC carbene. Since NHC carbenes are generally unstable to oxygen and water, the production must be performed under water-free and oxygen-free conditions using special equipment such as a glove box.
An object of the present invention is to provide a method for producing an amidate compound that does not require special equipment such as a glove box.

#### Solution to Problem

The present invention provides the following amidate compound, production method for the compound, blocking agent dissociation catalyst, and thermosetting resin composition.

1. A method for producing an amidate compound, the method comprising
reacting an imidazolium carboxylic acid salt represented by the following formula (1):

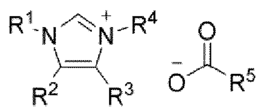 (1)

wherein

$R^1$ and $R^4$ are the same or different, and are each a $C_1$-$C_{20}$ hydrocarbon group optionally substituted with one or more heteroatoms,
$R^2$ and $R^3$ are the same or different, and are each a hydrogen atom or a $C_1$-$C_{20}$ hydrocarbon group optionally substituted with one or more heteroatoms, or $R^2$ and $R^3$, together with the carbon atoms to which they are attached, may form a ring structure, and $R^5$ is a hydrogen atom or a $C_1$-$C_{20}$ hydrocarbon group optionally substituted with one or more heteroatoms,
with
a polyisocyanate compound represented by the following formula (2):

$$A\left[NCO\right]_x \quad (2)$$

wherein
A is a residue obtained by removing isocyanate groups from at least one polyisocyanate selected from the group consisting of aliphatic polyisocyanates, alicyclic polyisocyanates, aromatic polyisocyanates, and aromatic aliphatic polyisocyanates, or a residue obtained by removing isocyanate groups from a modified isocyanate formed from at least one member selected from the group consisting of aliphatic polyisocyanates, alicyclic polyisocyanates, aromatic polyisocyanates, and aromatic aliphatic polyisocyanates, and
x is an integer of 2 or more and 20 or less,
wherein the amidate compound is represented by the following formula (3):

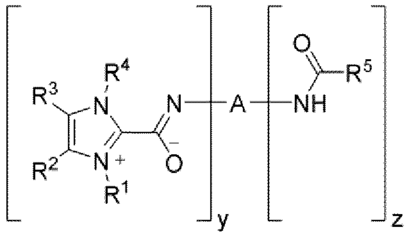

(3)

wherein y and z are each an integer of 1 or more and 19 or less, and the sum of y and z is 2 or more and 20 or less, and A, $R^1$, $R^2$, $R^3$, $R^4$, and $R^5$ are as defined above.

2. The method for producing an amidate compound according to Item 1, wherein the polyisocyanate compound represented by formula (2) is an aromatic polyisocyanate.

3. The method for producing an amidate compound according to Item 1, wherein the polyisocyanate compound represented by formula (2) is a dimeric or trimeric polyisocyanate formed from at least one member selected from the group consisting of 2,4-tolylene diisocyanate, 2,6-tolylene diisocyanate, 4,4'-diphenylmethane diisocyanate, and polymethylene polyphenyl polyisocyanate.

4. The method for producing an amidate compound according to Item 1, wherein the polyisocyanate compound represented by formula (2) is at least one polyisocyanate selected from the group consisting of 2,4-tolylene diisocyanate, 2,6-tolylene diisocyanate, 4,4'-diphenylmethane diisocyanate, and polymethylene polyphenyl polyisocyanate.

5. The method for producing an amidate compound according to any one of Items 1 to 4, wherein $R^2$ and $R^3$ are each a hydrogen atom.

6. An amidate compound represented by formula (3):

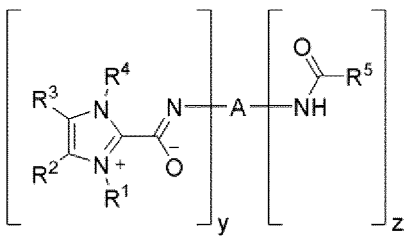

(3)

wherein

y and z are each an integer of 1 or more and 19 or less, and the sum of y and z is 2 or more and 20 or less, and
$R^1$ and $R^4$ are the same or different, and are each a $C_1$-$C_{20}$ hydrocarbon group optionally substituted with one or more heteroatoms,
$R^2$ and $R^3$ are the same or different, and are each a hydrogen atom or a $C_1$-$C_{20}$ hydrocarbon group optionally substituted with one or more heteroatoms, or $R^2$ and $R^3$, together with the carbon atoms to which they are

attached, may form a ring structure, and
$R^5$ is a hydrogen atom or a $C_1$-$C_{20}$ hydrocarbon group optionally substituted with one or more heteroatoms.

7. The amidate compound according to Item 6, wherein $R^2$ and $R^3$ are each a hydrogen atom.

8. The amidate compound according to Item 6, wherein $R^1$ and $R^4$ are each a $C_1$-$C_{20}$ alkyl group optionally substituted with one or more heteroatoms.

9. A blocking agent dissociation catalyst for blocked isocyanates, comprising the amidate compound of any one of Items 6 to 8.

10. A thermosetting resin composition comprising the amidate compound of any one of Items 6 to 8, a blocked isocyanate, and a compound having an isocyanate-reactive group.

11. A cured product obtained by curing the thermosetting resin composition of Item 10.

12. A method for producing a cured product, the method comprising the step of heating and curing the thermosetting resin composition of Item 10.

Advantageous Effects of Invention

The present invention is capable of providing a novel method for producing an amidate compound that does not require special equipment such as a glove box.

Further, the amidate compound represented by formula (3), which can be produced according to the present invention, is a novel compound and is useful as a blocking agent dissociation catalyst.

Description of Embodiments

Amidate Compound Represented by Formula (3) and Production Method Therefor

In the present invention, an amidate compound represented by formula (3) (hereinafter referred to as "the amidate compound (3)") is produced by reacting an imidazolium carboxylic acid salt represented by formula (1) (hereinafter referred to as "the imidazolium carboxylic acid salt (1)") with a polyisocyanate compound represented by formula (2) (hereinafter referred to as "the polyisocyanate compound (2)") optionally in the presence of a solvent. The reaction is usually performed by using the imidazolium carboxylic acid salt (1) and the polyisocyanate compound (2) such that c/2a = 0.5 to 2.0, wherein a is the number of moles of the imidazolium carboxylic acid salt (1), and c is the number of moles of the isocyanate groups in the polyisocyanate compound (2). When the imidazolium carboxylic acid salt (1) is produced by the production method for the imidazolium carboxylic acid salt (1) described later, a carboxylic acid (6) may remain in the imidazolium carboxylic acid salt (1). In such a case, the imidazolium carboxylic acid salt (1) and the polyisocyanate compound (2) are used such that c/(2a + b) = 0.5 to 2.0, wherein a is the number of moles of the imidazolium carboxylic acid salt (1), b is the number of moles of the carboxylic acid (6) remaining in the imidazolium carboxylic acid salt (1), and c is the number of moles of the isocyanate groups in the polyisocyanate compound (2).

The reaction usually proceeds favorably at a reaction temperature of -10°C or higher, and preferably 0°C to 150°C for a reaction time of 0.5 to 12 hours.

A solvent may or may not be used. Specific examples of solvents when used include aromatic hydrocarbons, such as toluene, benzene, and xylene; aliphatic or alicyclic hydrocarbons, such as methylcyclohexane, cyclohexane, hexane, heptane, and octane; halogenated hydrocarbons, such as dichloromethane, chloroform, carbon tetrachloride, and 1,2-dichloroethane; halogenated aromatic hydrocarbons, such as chlorobenzene and dichlorobenzene; ethers, such as diethyl ether, tetrahydrofuran, and 1,4-dioxane; and the like. Preferred are aromatic hydrocarbons and halogenated aromatic hydrocarbons, and particularly preferred is toluene. The solvents can be used as a mixture of two or more, if necessary.

The amount of solvent used is usually 50 parts by mass or less, and preferably 0.1 to 10 parts by mass, per part by mass of the imidazolium carboxylic acid salt (1).

The reaction may be performed, if necessary, in an inert gas atmosphere, such as nitrogen, argon, or helium, which do not affect the reaction.

After completion of the reaction, the amidate compound (3) can be obtained by removing the solvent by concentrating or filtering the reaction liquid, and may be purified by recrystallization, column separation, etc., if necessary.

In formula (1), $R^1$ and $R^4$ are each a $C_1$-$C_{20}$ hydrocarbon group optionally substituted with one or more heteroatoms, preferably, for example, a $C_1$-$C_{12}$ hydrocarbon group optionally substituted with one or more heteroatoms, and particularly preferably a $C_1$-$C_8$ hydrocarbon group optionally substituted with one or more heteroatoms. The hydrocarbon group is preferably an aliphatic hydrocarbon group, and more preferably an alkyl group. Examples of the $C_1$-$C_{20}$ hydrocarbon group optionally substituted with one or more heteroatoms include a methyl group, an ethyl group, a propyl group, an isopropyl group, a butyl group, a sec-butyl group, a tert-butyl group, a pentyl group, a hexyl group, a heptyl group, an octyl group, a 1,1,3,3-tetramethylbutyl group, a 1-ethylpentyl group, a 2-ethylhexyl group, a decyl group, a dodecyl group, a tetradecyl group, a hexadecyl group, an octadecyl group, an allyl group, a benzyl group, a cyclohexyl group, an adamantyl group, a phenyl group, a 2,6-diisopropylphenyl group, a 2,4,6-trimethylphenyl group, a 2-methoxyethyl group, a 2-ethoxyethyl group, 2-(dimethylamino)ethyl group, and the like. Preferred are a methyl group, an ethyl group, a propyl group, an isopropyl group, a butyl group, an octyl group, a dodecyl group, a cyclopentyl group, a cyclohexyl group, a 2-ethylhexyl group, a benzyl group, a phenyl group, and 2,4,6-trimethylphenyl group; and particularly preferred are a methyl group, an ethyl group, a butyl group, an octyl group, a 2-ethylhexyl group, and a benzyl group.

Examples of heteroatoms in $R^1$ and $R^4$ include nitrogen, oxygen, sulfur, and the like. When the hydrocarbon group is substituted with a heteroatom, such as oxygen, nitrogen, or sulfur, the hydrocarbon group has a group, such as -O-, -N<, -S-, or $-SO_2-$, and the hydrocarbon chain is interrupted by such a group. When the hydrocarbon group is substituted with a heteroatom, such as oxygen, nitrogen, or sulfur, it is preferred that the hydrocarbon group is substituted with oxygen and that the hydrocarbon chain is interrupted by an -O- group.

$R^2$ and $R^3$ are each a hydrogen atom or a $C_1$-$C_{20}$ hydrocarbon group optionally substituted with one or more heteroatoms, and preferably a hydrogen atom. The $C_1$-$C_{20}$ hydrocarbon group optionally substituted with one or more heteroatoms is preferably a $C_1$-$C_6$ hydrocarbon group optionally substituted with one or more heteroatoms, and particularly preferably a $C_1$-$C_4$ hydrocarbon group optionally substituted with one or more heteroatoms. Examples of the $C_1$-$C_{20}$ hydrocarbon group optionally substituted with one or more heteroatoms include a methyl group, an ethyl group, a propyl group, an isopropyl group, a butyl group, a sec-butyl group, a tert-butyl group, a pentyl group, a hexyl group, an octyl group, a 2-ethylhexyl group, a decyl group, a dodecyl group, a tetradecyl group, a hexadecyl group, an octadecyl group, an allyl group, a benzyl group, a cyclohexyl group, an adamantyl group, a phenyl group, a 2,6-diisopropylphenyl group, a 2,4,6-trimethylphenyl group, a 2-methoxyethyl group, a 2-ethoxyethyl group, a 2-(dimethylamino)ethyl group, and the like. Preferred are a methyl group, an ethyl group, a propyl group, an isopropyl group, a butyl group, a cyclopentyl group, a cyclohexyl group, a phenyl group, a 2-methoxyethyl group, a 2-ethoxyethyl group, and a 2-(dimethylamino)ethyl group; and particularly preferred are a methyl group, an ethyl group, a butyl group, a 2-methoxyethyl group, a 2-ethoxyethyl group, and a 2-(dimethylamino)ethyl group.

Examples of heteroatoms in $R^2$ and $R^3$ include nitrogen, oxygen, sulfur, and the like. When the hydrocarbon group is substituted with a heteroatom, such as oxygen, nitrogen, or sulfur, the hydrocarbon group has a group, such as -O-, -N<, -S-, or $-SO_2-$, and the hydrocarbon chain is interrupted by such a group. When the hydrocarbon group is substituted with a heteroatom, such as oxygen, nitrogen, or sulfur, it is preferred that the hydrocarbon group is substituted with oxygen and that the hydrocarbon chain is interrupted by an -O- group.

$R^2$ and $R^3$, together with the carbon atoms to which they are attached, may form a ring structure. When $R^2$ and $R^3$, together with the carbon atoms to which they are attached, form a ring structure, for example, a benzimidazolium ring structure as shown below can be formed:

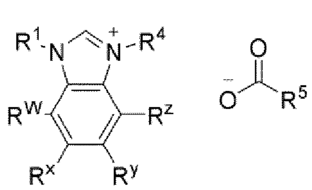
(1x)

wherein $R^1$, $R^4$, and $R^5$ are as defined above; and $R^w$, $R^x$, $R^y$, and $R^z$ are each a hydrogen atom or a $C_1$-$C_{20}$ hydrocarbon group.

Examples of the $C_1$-$C_{20}$ hydrocarbon group represented by $R^w$, $R^x$, $R^y$, or $R^z$ include a methyl group, an ethyl group, a propyl group, an isopropyl group, a butyl group, a sec-butyl group, a tert-butyl group, a pentyl group, a hexyl group, an octyl group, a 2-ethylhexyl group, a decyl group, a dodecyl group, a tetradecyl group, a hexadecyl group, an octadecyl group, an allyl group, a benzyl group, a cyclohexyl group, an adamantyl group, a phenyl group, a 2,6-diisopropylphenyl group, and a 2,4,6-trimethylphenyl group.

$R^5$ is a hydrogen atom or a $C_1$-$C_{20}$ hydrocarbon group optionally substituted with one or more heteroatoms, and preferably a $C_1$-$C_{20}$ hydrocarbon group optionally substituted with one or more heteroatoms. The $C_1$-$C_{20}$ hydrocarbon group optionally substituted with one or more heteroatoms is preferably a $C_1$-$C_8$ hydrocarbon group optionally substituted with one or more heteroatoms, and particularly preferably a $C_1$ or $C_2$ hydrocarbon group optionally substituted with one or more heteroatoms. The hydrocarbon group is preferably an aliphatic hydrocarbon group, and more preferably an alkyl

group. Examples of the $C_1$-$C_{20}$ hydrocarbon group optionally substituted with one or more heteroatoms include a methyl group, an ethyl group, a propyl group, an isopropyl group, a butyl group, a sec-butyl group, a tert-butyl group, a pentyl group, a hexyl group, a heptyl group, an octyl group, a 1-ethylpentyl group, a nonyl group, a 2-ethylhexyl group, a undecyl group, a tridecyl group, a pentadecyl group, a heptadecyl group, a vinyl group, an allyl group, a benzyl group, a cyclohexyl group, an adamantyl group, a phenyl group, a 2-methoxymethyl group, 2-ethoxymethyl group, a 2-(dimethylamino)methyl group, and the like. Preferred are a methyl group, an ethyl group, a propyl group, an isopropyl group, a butyl group, a heptyl group, a cyclohexyl group, a 1-ethylpentyl group, and a phenyl group, and particularly preferred are a methyl group, an ethyl group, a heptyl group, and a 1-ethylpentyl group.

Examples of heteroatoms in $R^5$ include nitrogen, oxygen, sulfur, and the like. When the hydrocarbon group is substituted with a heteroatom, such as oxygen, nitrogen, or sulfur, the hydrocarbon group has a group, such as -O-, -N<, -NH-, -S-, or - $SO_2$-, and the hydrocarbon chain is interrupted by such a group. When the hydrocarbon group is substituted with a heteroatom, such as oxygen, nitrogen, or sulfur, it is preferred that the hydrocarbon group is substituted with oxygen and that the hydrocarbon chain is interrupted by an -O- group. In another embodiment, when the hydrocarbon group is substituted with a heteroatom, such as oxygen, nitrogen, or sulfur, a hydrocarbon group having a group, such as -OH or -$NH_2$, may be formed.

Examples of the imidazolium carboxylic acid salt (1) include 1,3-dimethylimidazolium formate, 1-ethyl-3-methylimidazolium formate, 1-butyl-3-methylimidazolium formate, 1-methyl-3-octylimidazolium formate, 1-methyl-3-(1,1,3,3-tetramethylbutyl)imidazolium formate, 1-methyl-3-(2-ethylhexyl)imidazolium formate, 1-dodecyl-3-methylimidazolium formate, 1-methyl-3-octadecylimidazolium formate, 1-benzyl-3-methylimidazolium formate, 1,3-dibutylimidazolium formate, 1-butyl-3-ethylimidazolium formate, 1-butyl-3-octylimidazolium formate, 1-butyl-3-(1,1,3,3-tetramethylbutyl)imidazolium formate, 1-butyl-3-(2-ethylhexyl)imidazolium formate, 1-butyl-3-dodecylimidazolium formate, 1-butyl-3-octadecylimidazolium formate, 1-benzyl-3-butylimidazolium formate, 1,3-dioctylimidazolium formate, 1,3-bis(1,1,3,3-tetramethylbutyl)imidazolium formate, 1-ethyl-3-octylimidazolium formate, 1-ethyl-3-(1,1,3,3-tetramethylbutyl)imidazolium formate, 1-octyl-3-(2-ethylhexyl)imidazolium formate, 1-(1,1,3,3-tetramethylbutyl)-3-(2-ethylhexyl)imidazolium formate, 1-dodecyl-3-octylimidazolium formate, 1-dodecyl-3-(1,1,3,3-tetramethylbutyl)imidazolium formate, 1-octyl-3-octadecylimidazolium formate, 1-(1,1,3,3-tetramethylbutyl)-3-octadecylimidazolium formate, 1-benzyl-3-octylimidazolium formate, 1-benzyl-3-(1,1,3,3-tetramethylbutyl)imidazolium formate, 1,3-bis(2-ethylhexyl)imidazolium formate, 1-ethyl-3-(2-ethylhexyl)imidazolium formate, 1-(2-ethylhexyl)-3-dodecylimidazolium formate, 1-(2-ethylhexyl)-3-octadecylimidazolium formate, 1-benzyl-3-(2-ethylhexyl)imidazolium formate, 1,3-didodecylimidazolium formate, 1-dodecyl-3-octadecylimidazolium formate, 1-benzyl-3-dodecylimidazolium formate, 1,3-dioctadecylimidazolium formate, 1-benzyl-3-octadecylimidazolium formate, 1,3-dibenzylimidazolium formate;

1,3-dimethylimidazolium acetate, 1-ethyl-3-methylimidazolium acetate, 1-butyl-3-methylimidazolium acetate, 1-methyl-3-octylimidazolium acetate, 1-methyl-3-(1,1,3,3-tetramethylbutyl)imidazolium acetate, 1-methyl-3-(2-ethylhexyl)imidazolium acetate, 1-dodecyl-3-methylimidazolium acetate, 1-methyl-3-octadecylimidazolium acetate, 1-benzyl-3-methylimidazolium acetate, 1,3-dibutylimidazolium acetate, 1-butyl-3-ethylimidazolium acetate, 1-butyl-3-octylimidazolium acetate, 1-butyl-3-(1,1,3,3-tetramethylbutyl)imidazolium acetate, 1-butyl-3-(2-ethylhexyl)imidazolium acetate, 1-butyl-3-dodecylimidazolium acetate, 1-butyl-3-octadecylimidazolium acetate, 1-benzyl-3-butylimidazolium acetate, 1,3-dioctylimidazolium acetate, 1,3-bis(1,1,3,3-tetramethylbutyl)imidazolium acetate, 1-ethyl-3-octylimidazolium acetate, 1-ethyl-3-(1,1,3,3-tetramethylbutyl)imidazolium acetate, 1-octyl-3-(2-ethylhexyl)imidazolium acetate, 1-(1,1,3,3-tetramethylbutyl)-3-(2-ethylhexyl)imidazolium acetate, 1-dodecyl-3-octylimidazolium acetate, 1-dodecyl-3-(1,1,3,3-tetramethylbutyl)imidazolium acetate, 1-octyl-3-octadecylimidazolium acetate, 1-(1,1,3,3-tetramethylbutyl)-3-octadecylimidazolium acetate, 1-benzyl-3-octylimidazolium acetate, 1-benzyl-3-(1,1,3,3-tetramethylbutyl)imidazolium acetate, 1,3-bis(2-ethylhexyl)imidazolium acetate, 1-ethyl-3-(2-ethylhexyl)imidazolium acetate, 1-(2-ethylhexyl)-3-dodecylimidazolium acetate, 1-(2-ethylhexyl)-3-octadecylimidazolium acetate, 1-benzyl-3-(2-ethylhexyl)imidazolium acetate, 1,3-didodecylimidazolium acetate, 1-dodecyl-3-octadecylimidazolium acetate, 1-benzyl-3-dodecylimidazolium acetate, 1,3-dioctadecylimidazolium acetate, 1-benzyl-3-octadecylimidazolium acetate, 1,3-dibenzylimidazolium acetate;

1,3-dimethylimidazolium 2-ethylhexanoate, 1-ethyl-3-methylimidazolium 2-ethylhexanoate, 1-butyl-3-methylimidazolium 2-ethylhexanoate, 1-methyl-3-octylimidazolium 2-ethylhexanoate, 1-methyl-3-(1,1,3,3-tetramethylbutyl)imidazolium 2-ethylhexanoate, 1-methyl-3-(2-ethylhexyl)imidazolium 2-ethylhexanoate, 1-dodecyl-3-methylimidazolium 2-ethylhexanoate, 1-methyl-3-octadecylimidazolium 2-ethylhexanoate, 1-benzyl-3-methylimidazolium 2-ethylhexanoate, 1,3-dibutylimidazolium 2-ethylhexanoate, 1-butyl-3-ethylimidazolium 2-ethylhexanoate, 1-butyl-3-octylimidazolium 2-ethylhexanoate, 1-butyl-3-(1,1,3,3-tetramethylbutyl)imidazolium 2-ethylhexanoate, 1-butyl-3-(2-ethylhexyl)imidazolium 2-ethylhexanoate, 1-butyl-3-dodecylimidazolium 2-ethylhexanoate, 1-butyl-3-octadecylimidazolium 2-ethylhexanoate, 1-benzyl-3-butylimidazolium 2-ethylhexanoate, 1,3-dioctylimidazolium 2-ethylhexanoate, 1,3-bis(1,1,3,3-tetramethylbutyl)imidazolium 2-ethylhexanoate, 1-ethyl-3-octylimidazolium 2-ethylhexanoate, 1-ethyl-3-(1,1,3,3-tetramethylbutyl)imidazolium 2-ethylhexanoate, 1-octyl-3-(2-ethylhexyl)imidazolium 2-ethylhexanoate, 1-(1,1,3,3-tetramethylbutyl)-3-(2-ethylhexyl)imidazolium 2-ethylhexanoate, 1-dodecyl-3-octylimidazolium 2-ethylhexanoate, 1-dodecyl-3-(1,1,3,3-tetramethylbutyl)imidazolium 2-ethylhexanoate, 1-octyl-3-octadecylimidazolium 2-ethylhexanoate, 1-(1,1,3,3-tetramethylbutyl)-3-octadecylimidazolium 2-ethylhexanoate, 1-benzyl-3-octylimidazolium 2-ethylhexanoate, 1-benzyl-3-(1,1,3,3-tetramethylbutyl)imidazolium 2-ethylhexanoate, 1,3-bis(2-ethylhexyl)imidazolium 2-ethylhexanoate, 1-ethyl-3-(2-ethylhexyl)imidazolium 2-ethylhexanoate, 1-(2-ethylhexyl)-3-dodecylimidazolium 2-ethylhexanoate, 1-(2-ethylhexyl)-3-octadecylimidazolium 2-ethylhexanoate, 1-benzyl-3-(2-ethylhexyl)imidazolium 2-ethylhexanoate, 1,3-didodecylimidazolium 2-ethylhexanoate, 1-dodecyl-3-octadecylimidazolium 2-ethylhexanoate, 1-benzyl-3-dodecylimidazolium 2-ethylhexanoate, 1,3-dioctadecylimidazolium 2-ethylhexanoate, 1-benzyl-3-octadecylimidazolium 2-ethylhexanoate, 1,3-dibenzylimidazolium 2-ethylhexanoate; and

1,3-dimethylbenzimidazolium formate, 1,3-dimethylbenzimidazolium acetate, and 3-dimethylbenzimidazolium 2-ethylhexanoate.

The imidazolium carboxylic acid salt (1) is preferably 1,3-dimethylimidazolium acetate, 1-butyl-3-methylimidazolium acetate, 1-methyl-3-octylimidazolium acetate, 1-methyl-3-(1,1,3,3-tetramethylbutyl)imidazolium acetate, 1-methyl-3-(2-ethylhexyl)imidazolium acetate, 1-dodecyl-3-methylimidazolium acetate, 1,3-dibutylimidazolium acetate, 1-butyl-3-octylimidazolium acetate, 1-butyl-3-(1,1,3,3-tetramethylbutyl)imidazolium acetate, 1-butyl-3-(2-ethylhexyl)imidazolium acetate, 1-butyl-3-dodecylimidazolium acetate, 1,3-dioctylimidazolium acetate, 1-octyl-3-(1,1,3,3-tetramethylbutyl)imidazolium acetate, 1-octyl-3-(2-ethylhexyl)imidazolium acetate, 1-dodecyl-3-octylimidazolium acetate, 1-(1,1,3,3-tetramethylbutyl)-3-(2-ethylhexyl)imidazolium acetate, 1-dodecyl-3-(1,1,3,3-tetramethylbutyl)imidazolium acetate, bis(2-ethylhexyl)imidazolium acetate, 1-(2-ethylhexyl)-3-dodecylimidazolium acetate, 1,3-didodecylimidazolium acetate, 1,3-dimethylimidazolium 2-ethylhexanoate, 1-butyl-3-methylimidazolium 2-ethylhexanoate, 1-methyl-3-octylimidazolium 2-ethylhexanoate, 1-methyl-3-(1,1,3,3-tetramethylbutyl)imidazolium 2-ethylhexanoate, 1-methyl-3-(2-ethylhexyl)imidazolium 2-ethylhexanoate, 1-dodecyl-3-methylimidazolium 2-ethylhexanoate, 1,3-dibutylimidazolium 2-ethylhexanoate, 1-butyl-3-octylimidazolium 2-ethylhexanoate, 1-butyl-3-(1,1,3,3-tetramethylbutyl)imidazolium 2-ethylhexanoate, 1-butyl-3-(2-ethylhexyl)imidazolium 2-ethylhexanoate, 1-butyl-3-dodecylimidazolium 2-ethylhexanoate, 1,3-dioctylimidazolium 2-ethylhexanoate, 1-octyl-3-(1,1,3,3-tetramethylbutyl)imidazolium 2-ethylhexanoate, 1-octyl-3-(2-ethylhexyl)imidazolium 2-ethylhexanoate, 1-dodecyl-3-octylimidazolium 2-ethylhexanoate, 1-(1,1,3,3-tetramethylbutyl)-3-(2-ethylhexyl)imidazolium 2-ethylhexanoate, 1-dodecyl-3-(1,1,3,3-tetramethylbutyl)imidazolium 2-ethylhexanoate, bis(2-ethylhexyl)imidazolium 2-ethylhexanoate, 1-(2-ethylhexyl)-3-dodecylimidazolium 2-ethylhexanoate, and 1,3-didodecylimidazolium 2-ethylhexanoate. More preferred are bis(2-ethylhexyl)imidazolium acetate and bis(2-ethylhexyl)imidazolium 2-ethylhexanoate.

The imidazolium carboxylic acid salt (1) may be a commercial product. The imidazolium carboxylic acid salt (1) may be a salt obtained by a known method or a salt produced by a method explained below.

The imidazolium carboxylic acid salt represented by formula (1) is obtained by reacting a dicarbonyl compound represented by the following formula (4), primary amine compounds represented by the following formulas (5a) and (5b), formaldehyde, and a carboxylic acid represented by the following formula (6).

Formula (4):

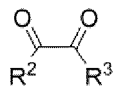 (4)

wherein $R^2$ and $R^3$ are as defined above.

Formula (5a):

$$R^1\text{-}NH_2 \qquad (5a),$$

wherein $R^1$ is as defined above.

Formula (5b):

$$R^4\text{-}NH_2 \qquad (5b),$$

wherein $R^4$ is as defined above.

Formula (6):

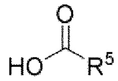 (6)

wherein $R^5$ is as defined above.

Preferable examples of the dicarbonyl compound represented by formula (4) (hereinafter referred to as "the dicarbonyl compound (4)") include glyoxal, diacetyl, 3,4-hexanedione, 2,3-pentanedione, 2,3-heptanedione, 5-methyl-2,3-hexanedione, 3-methyl-2,3-cyclopentanedione, 1,2-cyclohexanedione, 1-phenyl-1,2-propanedione, and dibenzoyl; more preferably glyoxal and diacetyl; and still more preferably glyoxal.

The primary amine compound represented by formula (5a) (hereinafter referred to as "the primary amine compound (5a)") and the primary amine compound represented by formula (5b) (hereinafter referred to as "the primary amine compound (5b)") are at least one primary amine compound selected from the group consisting of methylamine, ethylamine, propylamine, isopropylamine, butylamine, tert-butylamine, hexylamine, octylamine, 1,1,3,3-tetramethylbutylamine, 2-ethylhexylamine, dodecylamine, tetradecylamine, hexadecylamine, octadecylamine, 2-methoxyethylamine, 2-ethoxyethylamine, 3-methoxypropylamine, 3-ethoxypropylamine, 3-propoxypropylamine, 3-isopropoxypropylamine, 3-butoxypropylamine, 3-(2-ethylhexyloxy)propylamine, allylamine, benzylamine, aniline, 2,6-diisopropylaniline, and 2,4,6-trimethylaniline; preferably methylamine, ethylamine, butylamine, hexylamine, octylamine, 1,1,3,3-tetramethylbutylamine, 2-ethylhexylamine, dodecylamine, octadecylamine, and benzylamine; and more preferably methylamine, butylamine, octylamine, and 2-ethylhexylamine.

Preferable examples of the carboxylic acid represented by formula (6) (hereinafter referred to as "the carboxylic acid (6)") include carboxylic acids, such as formic acid, acetic acid, propionic acid, butyric acid, pentanoic acid, hexanoic acid, heptanoic acid, octanoic acid, 2-ethylhexanoic acid, capric acid, lauric acid, tetradecylic acid, palmitic acid, octadecylic acid, cyclohexanoic acid, ethoxyacetic acid, propoxyacetic acid, 2-(2-methoxyethoxy)acetic acid, 2-(2-ethoxyethoxy)acetic acid, 2-(2-propoxyethoxy)acetic acid, 3-methoxypropanoic acid, 3-ethoxypropanoic acid, 3-(2-methoxyethoxy)propanoic acid, 3-(2-ethoxyethoxy)propanoic acid, 3-(2-propoxyethoxy)propanoic acid, 3-(3-methoxypropoxy)propanoic acid, 3-(3-ethoxypropoxy)propanoic acid, 3-(3-propoxypropoxy)propanoic acid, oleic acid, linoleic acid, sorbic acid, benzoic acid, phthalic acid, isophthalic acid, terephthalic acid, lactic acid, salicylic acid, and trifluoroacetic acid. More preferred are formic acid, acetic acid, propionic acid, butyric acid, pentanoic acid, hexanoic acid, heptanoic acid, octanoic acid, and 2-ethylhexanoic acid, and particularly preferred are acetic acid and 2-ethylhexanoic acid.

As the dicarbonyl compound (4), an aqueous solution or an alcohol solution, such as methanol or butanol, may be used as is.

The amounts of the primary amine compound (5a) and the primary amine compound (5b) (the primary amine compound (5a) and the primary amine compound (5b) are hereinafter collectively referred to as "the amine compounds (5)") used are usually such that the amount of the amine compounds (5) is 0.1 to 10 mol, and preferably 0.5 to 3 mol, per mole of the dicarbonyl compound (4). When the amine compounds (5) are allowed to react in an amount of 2 mol per mole of the dicarbonyl compound (4), 1 mol of the imidazolium carboxylic acid salt (1) is obtained. For example, when the amine compounds (5) are used in an amount of less than 2 mol, the dicarbonyl compound (4) (starting material) and a polymer of the dicarbonyl compound (4) are present in addition to the desired imidazolium carboxylic acid salt (1). When the amine compounds (5) are used in an amount of more than 2 mol per mole of the dicarbonyl compound (4), an excess amount of the amine compounds (5) is present in addition to the desired imidazolium carboxylic acid salt (1). The amidate compound (3) can be obtained even when the imidazolium carboxylic acid salt (1) present together with such a compound other than the imidazolium carboxylic acid salt (1) is used.

The ratio of the primary amine compound (5a) to the primary amine compound (5b) is not particularly limited, and is such that primary amine compound (5a):primary amine compound (5b) = 0:100 to 100:0 (molar ratio). When primary amine compound (5a):primary amine compound (5b) = 0:100 or primary amine compound (5a):primary amine compound (5b) = 100:0, $R^1 = R^4$. When $R^1$ is not equal to $R^4$, that is, when primary amine compound (5a):primary amine compound (5b) is not 0:100 or 100:0, the compound of formula (1) can be a mixture of compounds represented by the following formulas (1-1), (1-2), and (1-3).

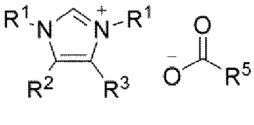 (1-1)

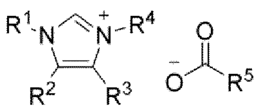
(1-2)

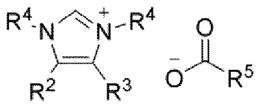
(1-3)

In formulas (1-1), (1-2), and (1-3), $R^1$, $R^2$, $R^3$, $R^4$, and $R^5$ are as defined above.

The ratio of the compound represented by formula (1-1), the compound represented by formula (1-2), and the compound represented by formula (1-3) in the mixture varies depending on the ratio of the primary amine compound (5a) to the primary amine compound (5b) used in the reaction. The compound represented by formula (1-1), the compound represented by formula (1-2), and the compound represented by formula (1-3) are all encompassed by the imidazolium carboxylic acid salt (1).

As the formaldehyde, an aqueous solution or an alcohol solution, such as methanol or butanol, may be used as is. The amount of formaldehyde used is generally 0.1 to 10 mol, and preferably 0.5 to 5.0 mol, per mole of the dicarbonyl compound (6) .

The amount of the carboxylic acid (6) used is generally 0.1 to 10 mol, preferably 0.5 to 2 mol, and more preferably 1 to 1.5 mol, per mole of the dicarbonyl compound (4).

The optimal reaction temperature varies depending on the starting materials, solvents, etc. used, but is generally -10°C or higher, and preferably 0°C to 100°C. The reaction time is not particularly limited, and is preferably 0.5 to 48 hours.

A solvent may or may not be used. When a solvent is used, the solvent used is not particularly limited, as long as it does not affect the reaction. Specific examples of solvents include aromatic hydrocarbons, such as toluene, benzene, and xylene; aliphatic or alicyclic hydrocarbons, such as methylcyclohexane, cyclohexane, hexane, heptane, and octane; halogenated hydrocarbons, such as dichloromethane, chloroform, carbon tetrachloride, and 1,2-dichloroethane; ethers, such as diethyl ether, tetrahydrofuran, and 1,4-dioxane; lower alcohols, such as methanol and ethanol; N,N-dimethylformamide, acetonitrile, water, and the like. Preferred are aromatic hydrocarbons, lower alcohols, and water; and particularly preferred are toluene and water. The solvents can be used as a mixture of two or more, if necessary.

The amount of solvent used is generally 50 parts by mass or less, and preferably 0.1 to 10 parts by mass, per part by mass of the dicarbonyl compound (4).

The reaction may be performed, if necessary, in an inert gas atmosphere, such as nitrogen, argon, or helium, which do not affect the reaction.

After completion of the reaction, the imidazolium carboxylic acid salt (1) can be isolated, for example, by removing impurities (e.g., unreacted starting materials) by washing with an organic solvent, or concentrating the reaction liquid, and may be purified by recrystallization etc., if necessary.

During the production of the imidazolium carboxylic acid salt (1), the carboxylic acid (6) used in excess of the stoichiometric amount may remain in the imidazolium carboxylic acid salt (1). In this case, the remaining carboxylic acid (6) can be transformed into the corresponding ester compound by reacting with a carbonic acid ester.

Specific examples of the carbonic acid ester include dialkyl carbonates, such as dimethyl carbonate, diethyl carbonate, dipropyl carbonate, dibutyl carbonate, dipentyl carbonate, and dihexyl carbonate; and cyclic alkylene carbonates, such as ethylene carbonate, propylene carbonate, and butylene carbonate. Preferred are dimethyl carbonate, diethyl carbonate, dipropyl carbonate, and dibutyl carbonate; and particularly preferred is dimethyl carbonate.

The amount of the carbonic acid ester used is generally 1 mol or more, and preferably 1 to 6 mol, per mole of the remaining carboxylic acid (6). When water is contained in the imidazolium carboxylic acid salt (1) together with the carboxylic acid (6), water reacts with the carbonic acid ester; thus, it is preferable to use the carbonic acid ester in an amount of generally 1 mol or more, and preferably an excess of 1 to 6 mol, per mole of the total of the carboxylic acid (6) and water contained in the imidazolium carboxylic acid salt (1). The carboxylic acid (6) can be transformed into the corresponding ester compound at a reaction temperature of 30 to 100°C for a reaction time of 1 to 8 hours. For example, washing the transformed ester compound with an organic solvent or concentration of the reaction liquid results in the removal of the carboxylic acid (6) contained in the imidazolium carboxylic acid salt (1). Even when the imidazolium carboxylic acid salt (1) that contains the ester compound transformed with the use of a carbonic acid ester is used, the target amidate compound (3) can be obtained according to the production method of the present invention.

Next, the polyisocyanate compound (2) will be explained.

In formula (2), A is any one of the following residues (i) to (v) (which may be simply referred to below as "the residue").

(i) A residue obtained by removing isocyanate groups from an aliphatic polyisocyanate
(ii) A residue obtained by removing isocyanate groups from an alicyclic polyisocyanate
(iii) A residue obtained by removing isocyanate groups from an aromatic polyisocyanate
(iv) A residue obtained by removing isocyanate groups from an aromatic aliphatic polyisocyanate
(v) A residue obtained by removing isocyanate groups from a modified isocyanate formed from at least one member selected from the group consisting of aliphatic polyisocyanates, alicyclic polyisocyanates, aromatic polyisocyanates, and aromatic aliphatic polyisocyanates

Aliphatic polyisocyanates, alicyclic polyisocyanates, aromatic polyisocyanates, aromatic aliphatic polyisocyanates, or modified isocyanates thereof are compounds having isocyanate groups, and the residue A itself represents a moiety other than the isocyanate groups of an aliphatic polyisocyanate, alicyclic polyisocyanate, aromatic polyisocyanate, aromatic aliphatic polyisocyanate, or modified isocyanate thereof. The residue A is usually an x-valent hydrocarbon group optionally substituted with one or more substituents other than isocyanate groups, and preferably comprises an x-valent hydrocarbon group optionally substituted with one or more heteroatoms or one or more halogen atoms. In this case, the hydrocarbon group preferably has 1 to 100 carbon atoms. In another embodiment, it is preferred that the residue does not have an active hydrogen group, such as a hydroxyl group or an amino group. The x in the x-valent above is the same number as x in formula (2).
Examples of the substituents of the x-valent hydrocarbon group optionally substituted with one or more substituents other than isocyanate groups represented by the residue A include halogen atoms, such as fluorine, chlorine, bromine, and iodine, and dialkylamino groups, alkoxy groups, aryloxy groups, a nitro group, a cyano group, a sulfonyl group, (monoalkylamino)carbonylamino groups, and (dialkylamino)carbonylamino groups.
The hydrocarbon group of the residue A may be substituted with one or more heteroatoms, such as oxygen, nitrogen, and sulfur. When the hydrocarbon group of the residue A is substituted with a heteroatom, such as oxygen, nitrogen, or sulfur, the hydrocarbon group has a group, such as -O-, -N<, -S-, or $-SO_2-$, and the hydrocarbon chain is interrupted by such a group.
Examples of the substituted or unsubstituted x-valent hydrocarbon group include alkylene groups, such as an ethylene group, an n-propylene group, an n-butylene group, an n-pentylene group, an n-hexylene group, an n-heptylene group, an n-octylene group, an n-nonylene group, an n-decylene group, an n-dodecylene group, an n-octadecylene group, cyclohexylene, cyclohexane-1,2-diylbismethylene, and a cyclohexane-1,4-diylbismethylene group; arylene groups, such as a p-phenylene group, an m-phenylene group, a 2-methyl-m-phenylene group, a 4-methyl-m-phenylene group, a 5-methyl-m-phenylene group, and a naphthylene group; arylalkylene groups, such as a phenylethylene group, a 1-phenylpropylene group, a 2-phenylpropylene group, a 1-phenylbutylene group, a 2-phenylbutylene group, and a naphthylethylene group; alkylenearylene groups obtained by suitably combining the above alkylene groups and arylene groups, such as a methylene diphenylene group and a polymethylene polyphenylene group; and the like.
Preferable examples of the residue A include the following groups.

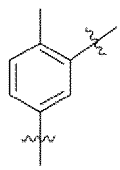 , 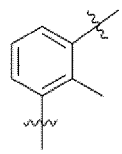 , 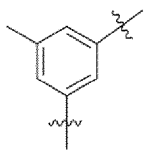 , 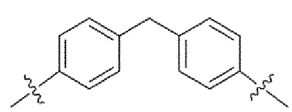 ,

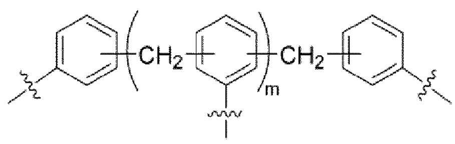

wherein m is an integer of 0 to 4.
x is an integer of 2 or more and 20 or less, preferably 2 to 6, more preferably 2 to 4, and particularly preferably 2 or 3.

Examples of the polyisocyanate compound (2) include aliphatic polyisocyanates, alicyclic polyisocyanates, aromatic polyisocyanates, aromatic aliphatic polyisocyanates, and modified isocyanates thereof. The polyisocyanate compound (2) may be monomeric, dimeric, trimeric, or multimeric.
Examples of aliphatic polyisocyanates include aliphatic diisocyanates, lysine triisocyanate, 4-isocyanatomethyl-1,8-octamethylene diisocyanate, and bis(2-isocyanatoethyl)2-isocyanato glutarate.
The aliphatic diisocyanates are preferably those having 4 to 30 carbon atoms. Examples include 1,4-tetramethylene diisocyanate, 1,6-hexamethylene diisocyanate (hereinafter referred to as "HDI"), 2,2,4-trimethylhexamethylene diisocyanate, 2,4,4-trimethylhexamethylene diisocyanate, lysine diisocyanate, and the like. Preferred is HDI. The aliphatic polyisocyanates may be used singly or in a combination of two or more.

Preferable examples of alicyclic polyisocyanates include those having 8 to 30 carbon atoms. Specific examples include 1,3-bis(isocyanatomethyl)cyclohexane, 1,4-bis(isocyanatomethyl)cyclohexane, 3-isocyanatomethyl-3,3,5-trimethylcyclohexyl isocyanate (hereinafter referred to as "IPDI"), bis(4-isocyanatocyclohexyl)methane, norbornane diisocyanate, dimer acid diisocyanate, and the like. Preferred is IPDI. The alicyclic polyisocyanates may be used singly or in a combination of two or more.

Examples of aromatic polyisocyanates include aromatic diisocyanates and polymethylene polyphenyl polyisocyanate (hereinafter referred to as "polymeric MDI"). Examples of aromatic diisocyanates include 2,4'-diphenylmethane diisocyanate, 4,4'-diphenylmethane diisocyanate, crude diphenylmethane diisocyanate, 1,4-phenylene diisocyanate, 2,4-tolylene diisocyanate, 2,6-tolylene diisocyanate, 3,3'-dimethyl-4,4'-diisocyanatobiphenyl, 3,3'-dimethyl-4,4'-diisocyanatodiphenylmethane, 1,5-naphthylene diisocyanate, and the like. The aromatic polyisocyanates may be used singly or in a combination of two or more. Preferred are 2,4-tolylene diisocyanate, 2,6-tolylene diisocyanate, 4,4'-diphenylmethane diisocyanate, and polymeric MDI, from the standpoint of higher industrial availability.

Examples of aromatic aliphatic polyisocyanates include 1,3-xylylene diisocyanate, 1,4-xylylene diisocyanate, $\alpha,\alpha,\alpha',\alpha'$-tetramethylxylylene diisocyanate, and the like. The aromatic aliphatic polyisocyanates may be used singly or in a combination of two or more.

Among these polyisocyanate compounds, preferred are aromatic polyisocyanates, and more preferred are 2,4-tolylene diisocyanate, 2,6-tolylene diisocyanate, 4,4'-diphenylmethane diisocyanate, and polymeric MDI.

Examples of modified isocyanates include 2- to 20-mer oligomers of the above polyisocyanates produced by forming a biuret bond, urea bond, isocyanurate bond, uretdione bond, urethane bond, allophanate bond, oxadiazintrione bond, or the like. Polyisocyanates with biuret bonds are obtained by reacting a biuretting agent, such as water, tert-butanol, or urea, with a polyisocyanate in a molar ratio of biuretting agent/isocyanate groups in the polyisocyanate of about 1/2 to about 1/100, followed by removal of unreacted polyisocyanate by purification. Polyisocyanates with isocyanurate bonds are obtained, for example, by performing a cyclic trimerization reaction with a catalyst or the like, and stopping the reaction when the conversion reaches about 5 to about 80 mass%, followed by removal of unreacted polyisocyanate by purification.

Polyisocyanate compounds with urethane bonds encompassed by modified isocyanates are obtained by, for example, reacting a 2- to 6-valent alcohol compound, such as trimethylolpropane, with a polyisocyanate in a molar ratio of hydroxyl groups in the alcohol compound/isocyanate groups in the polyisocyanate of about 1/2 to about 1/100, followed by removal of unreacted polyisocyanate by purification. Removal of unreacted polyisocyanate by purification is not always necessary. The modified isocyanate compound is preferably a dimeric or trimeric polyisocyanate formed from at least one member selected from the group consisting of 2,4-tolylene diisocyanate, 2,6-tolylene diisocyanate, 4,4'-diphenylmethane diisocyanate, and polymethylene polyphenyl polyisocyanate.

Formula (3)

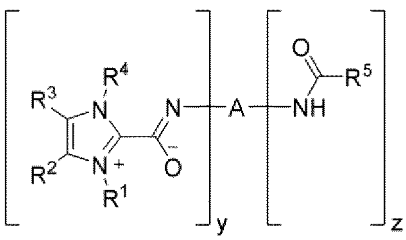

(3)

In formula (3), y and z are each an integer of 1 or more and 19 or less, and the sum of y and z is 2 or more and 20 or less. It is preferred that y and z are each 1 to 5, and that the sum of y and z is 2 to 6; it is more preferred that y and z are each 1 to 3, and that the sum of y and z is 2 to 4; and it is particularly preferred that y and z are each 1 or 2, and that the sum of y and z is 2 or 3.

A, $R^1$, $R^2$, $R^3$, $R^4$, and $R^5$ are as defined above.

When the amidate compound (3) is an isomer, such as an enantiomer, a stereoisomer, or a regioisomer, the amidate compound (3) includes a mixture of any isomers, unless the isomer is specified. For example, when the amidate compound (3) is an enantiomer, the amidate compound (3) also includes enantiomers divided from the racemic form. These isomers can be obtained as single compounds by conventionally known separation methods (concentration, solvent extraction, column chromatography, recrystallization, etc.).

Examples of the amidate compound (3) of the present invention include the following. In the following specific examples, Et represents an ethyl group, Bu represents an n-butyl group, Hept represents an n-heptyl group, Oct represents an n-octyl group, 1-EtPent represents a 1-ethylpentyl group, and 2-EtHex represents a 2-ethylhexyl group.

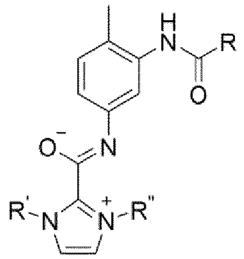

| R | R' | R" | | R | R' | R" | |
|---|---|---|---|---|---|---|---|
| $CH_3$ | $CH_3$ | $CH_3$ | (3-1-1) | Hept | Oct | Oct | (3-1-10) |
| $CH_3$ | $CH_3$ | Oct | (3-1-2) | Hept | Oct | 2-EtHex | (3-1-11) |
| $CH_3$ | $CH_3$ | 2-EtHex | (3-1-3) | Hept | 2-EtHex | 2-EtHex | (3-1-12) |
| $CH_3$ | Oct | Oct | (3-1-4) | 1-EtPent | $CH_3$ | $CH_3$ | (3-1-13) |
| $CH_3$ | Oct | 2-EtHex | (3-1-5) | 1-EtPent | $CH_3$ | Oct | (3-1-14) |
| $CH_3$ | 2-EtHex | 2-EtHcx | (3-1-6) | 1-EtPent | $CH_3$ | 2-EtHcx | (3-1-15) |
| Hept | $CH_3$ | $CH_3$ | (3-1-7) | 1-EtPent | Oct | Oct | (3-1-16) |
| Hept | $CH_3$ | Oct | (3-1-8) | 1-EtPent | Oct | 2-EtHex | (3-1-17) |
| Hept | $CH_3$ | 2-EtHex | (3-1-9) | 1-EtPent | 2-EtHex | 2-EtHex | (3-1-18) |

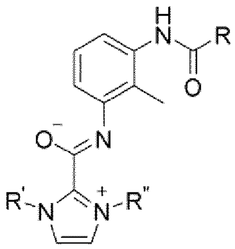

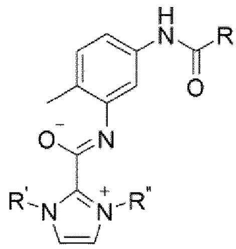

| R | R' | R' ' | | R | R' | R' ' | |
|---|---|---|---|---|---|---|---|
| $CH_3$ | $CH_3$ | $CH_3$ | (3 2 1) | $CH_3$ | $CH_3$ | $CH_3$ | (3 3 1) |
| $CH_3$ | $CH_3$ | Oct | (3-2-2) | $CH_3$ | $CH_3$ | Oct | (3-3-2) |
| $CH_3$ | $CH_3$ | 2-EtHex | (3-2-3) | $CH_3$ | $CH_3$ | 2-EtHex | (3-3-3) |
| $CH_3$ | Oct | Oct | (3-2-4) | $CH_3$ | Oct | Oct | (3-3-4) |
| $CH_3$ | Oct | 2-EtHex | (3-2-5) | $CH_3$ | Oct | 2-EtHex | (3-3-5) |
| $CH_3$ | 2-EtHex | 2-EtHex | (3-2-6) | $CH_3$ | 2-EtHex | 2-EtHex | (3-3-6) |
| Hept | $CH_3$ | $CH_3$ | (3-2-7) | Hept | $CH_3$ | $CH_3$ | (3-3--7) |
| Hept | $CH_3$ | Oct | (3-2-8) | Hept | $CH_3$ | Oct | (3-3-8) |
| Hept | $CH_3$ | 2-EtHex | (3-2-9) | Hept | $CH_3$ | 2-EtHex | (3-3-9) |
| Hept | Oct | Oct | (3-2-10) | Hept | Oct | Oct | (3-3-10) |
| Hept | Oct | 2-EtHex | (3-2-11) | Hept | Oct | 2-EtHex | (3-3-11) |
| Hept | 2-EtHex | 2-EtHex | (3 2 12) | Hept | 2-EtHex | 2-EtHex | (3-3-12) |
| 1-EtPent | $CH_3$ | $CH_3$ | (3-2-13) | 1-EtPent | $CH_3$ | $CH_3$ | (3-3-13) |
| 1-EtPent | $CH_3$ | Oct | (3-2-14) | 1-EtPent | $CH_3$ | Oct | (3-3-14) |
| 1-EtPent | $CH_3$ | 2-EtHex | (3-2-15) | 1-EtPent | $CH_3$ | 2-EtHex | (3-3-15) |
| 1-EtPent | Oct | Oct | (3-2-16) | 1-EtPent | Oct | Oct | (3-3-16) |
| 1-EtPent | Oct | 2-EtHex | (3-2-17) | 1-EtPent | Oct | 2-EtHex | (3-3-17) |

(continued)

| 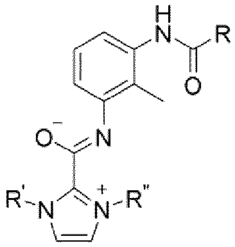 | | | | 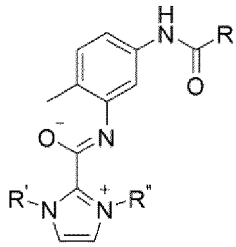 | | | |
|---|---|---|---|---|---|---|---|
| R | R' | R'' | | R | R' | R'' | |
| 1-EtPent | 2-EtHex | 2-EtHex | (3-2-18) | 1-EtPent | 2-EtHex | 2-EtHex | (3-3-18) |

| 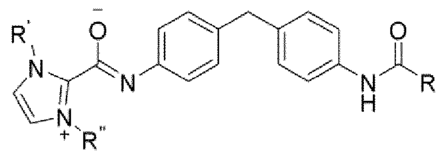 | | | | 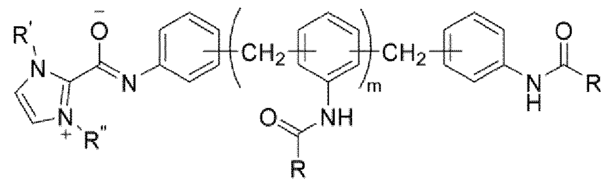 | | | |
|---|---|---|---|---|---|---|---|
| R | R' | R" | | R | R' | R" | |
| $CH_3$ | $CH_3$ | $CH_3$ | (3-4-1) | $CH_3$ | $CH_3$ | $CH_3$ | (3-5-1) |
| $CH_3$ | $CH_3$ | Oct | (3-4-2) | $CH_3$ | $CH_3$ | Oct | (3-5-2) |
| $CH_3$ | $CH_3$ | 2-EtHex | (3-4-3) | $CH_3$ | $CH_3$ | 2-EtHex | (3-5-3) |
| $CH_3$ | Oct | Oct | (3-4-4) | $CH_3$ | Oct | Oct | (3-5-4) |
| $CH_3$ | Oct | 2-EtHex | (3-4-5) | $CH_3$ | Oct | 2-EtHex | (3-5-5) |
| $CH_3$ | 2-EtHex | 2-EtHex | (3-4-6) | $CH_3$ | 2-EtHex | 2-EtHex | (3-5-6) |
| Hept | $CH_3$ | $CH_3$ | (3-4-7) | Hept | $CH_3$ | $CH_3$ | (3-5-7) |
| Hept | $CH_3$ | Oct | (3-4-8) | Hept | $CH_3$ | Oct | (3-5-8) |
| Hept | $CH_3$ | 2-EtHex | (3-4-9) | Hept | $CH_3$ | 2-EtHex | (3-5-9) |
| Hept | Oct | Oct | (3-4-10) | Hept | Oct | Oct | (3-5-10) |
| Hept | Oct | 2-EtHex | (3-4-11) | Hept | Oct | 2-EtHex | (3-5-11) |
| Hept | 2-EtHex | 2-EtHex | (3-4-12) | Hept | 2-EtHex | 2-EtHex | (3-5-12) |
| 1-EtPent | $CH_3$ | $CH_3$ | (3-4-13) | 1-EtPent | $CH_3$ | $CH_3$ | (3-5-13) |
| 1-EtPent | $CH_3$ | Oct | (3-4-14) | 1-EtPent | $CH_3$ | Oct | (3-5-14) |
| 1-EtPent | $CH_3$ | 2-EtHex | (3-4-15) | 1-EtPent | $CH_3$ | 2-EtHex | (3-5-15) |
| 1-EtPent | Oct | Oct | (3-4-16) | 1 EtPent. | Oct | Oct | (3-5-16) |
| 1-EtPent | Oct | 2-EtHex | (3-4-17) | 1-EtPent | Oct | 2-EtHex | (3-5-17) |
| 1-EtPent | 2-EtHex | 2-EtHex | (3-4-18) | 1-EtPent | 2-EtHex | 2-EtHex | (3-5-18) |

In formulas (3-5-1) to (3-5-18), m is an integer of 0 to 4.

| 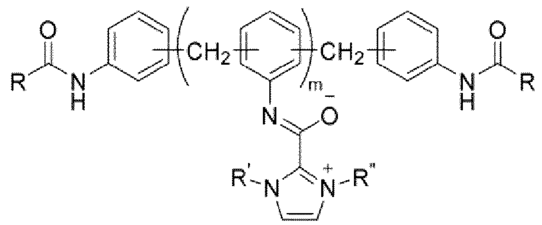 | | | | 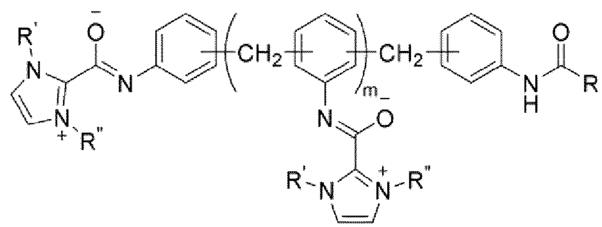 | | | |
|---|---|---|---|---|---|---|---|
| R | R' | R'' | | R | R' | R'' | |
| $CH_3$ | $CH_3$ | $CH_3$ | (3-6-1) | $CH_3$ | $CH_3$ | $CH_3$ | (3-7-1) |
| $CH_3$ | $CH_3$ | Oct | (3-6-2) | $CH_3$ | $CH_3$ | Oct | (3-7-2) |
| $CH_3$ | $CH_3$ | 2-EtHex | (3-6-3) | $CH_3$ | $CH_3$ | Z--EtHex | (3-7-3) |
| $CH_3$ | Oct | Oct | (3-6-4) | $CH_3$ | Oct | Oct | (3-7-4) |
| $CH_3$ | Oct | 2-EtHex | (3-6-5) | $CH_3$ | Oct | 2-EtHex | (3-7-5) |
| $CH_3$ | 2-EtHex | 2-EtHex | (3-6-6) | $CH_3$ | 2-EtHex | 2-EtHex | (3-7-6) |
| Hept | $CH_3$ | $CH_3$ | (3-6-7) | Hept | $CH_3$ | $CH_3$ | (3-7-7) |
| Hept | $CH_3$ | Oct | (3-6-8) | Hept | $CH_3$ | Oct | (3-7-8) |
| Hept | $CH_3$ | 2-EtHex | (3-6-9) | Hept | $CH_3$ | 2-EtHex | (3-7-9) |
| Hept | Oct | Oct | (3-6-10) | Hept | Oct | Oct | (3-7-10) |
| Hept | Oct | 2-EtHex | (3-6-11) | Hept | Oct | 2--EtHex | (3-7-11) |
| Hept | 2-EtHex | 2-EtHex | (3-6-12) | Hept | 2-EtHex | 2-EtHex | (3-7-12) |
| 1-EtPent | $CH_3$ | $CH_3$ | (3-6--13) | 1-EtPent | $CH_3$ | $CH_3$ | (3-7-13) |
| 1-EtPent | $CH_3$ | Oct | (3-6-14) | 1-EtPent | $CH_3$ | Oct | (3-7-14) |
| 1-EtPent | $CH_3$ | 2-EtHex | (3-6-15) | 1-EtPent | $CH_3$ | 2-EtHex | (3-7-15) |
| 1-EtPent | Oct | Oct | (3-6-16) | 1-EtPent | Oct | Oct | (3-7-16) |
| 1-EtPent | Oct | 2-EtHex | (3-6-17) | 1-EtPent | Oct | 2-EtHex | (3-7-17) |
| 1-EtPent | 2-EtHex | 2-EtHex | (3-6-18) | 1-EtPent | 2-EtHex | 2-EtHex | (3-7-18) |

In formulas (3-6-1) to (3-6-18) and (3-7-1) to (3-7-18), m is an integer of 0 to 4.

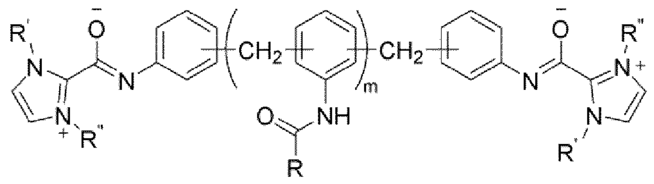

| R | R' | R'' | | R | R' | R'' | |
|---|---|---|---|---|---|---|---|
| $CH_3$ | $CH_3$ | $CH_3$ | (3-8-1) | Hept | Oct | Oct | (3-8-10) |
| $CH_3$ | $CH_3$ | Oct | (3-8-2) | Hept | Oct | 2-EtHex | (3-8-11) |
| $CH_3$ | $CH_3$ | 2-EtHex | (3-8-3) | Hept | 2-EtHex | 2-EtHex | (3-8-12) |
| $CH_3$ | Oct | Oct | (3-8-4) | 1-EtPent | $CH_3$ | $CH_3$ | (3-8-13) |
| $CH_3$ | Oct | 2-EtHex | (3-8-5) | 1-EtPent | $CH_3$ | Oct | (3-8-14) |
| $CH_3$ | 2-EtHex | 2-EtHex | (3-8-6) | 1-EtPent | $CH_3$ | 2-EtHex | (3-8-15) |
| Hept | $CH_3$ | $CH_3$ | (3-8-7) | 1-EtPent | Oct | Oct | (3-8-16) |
| Hept | $CH_3$ | Oct | (3-8-8) | 1-EtPent | Oct | 2-EtHex | (3-8-17) |
| Hept | $CH_3$ | 2-EtHex | (3-8-9) | 1-EtPent | 2--EtHex | 2-EtHex | (3-8-18) |

In formulas (3-8-1) to (3-8-18), m is an integer of 0 to 4.

The amidate compound (3) is preferably a compound represented by formula (3-1-4), (3-1-6), (3-1-10), (3-1-12), (3-1-16), (3-1-18), (3-2-4), (3-2-6), (3-2-10), (3-2-12), (3-2-16), (3-2-18), (3-3-4), (3-3-6), (3-3-10), (3-3-12), (3-3-16), (3-3-18), (3-4-4), (3-4-6), (3-4-10), (3-4-12), (3-4-16), (3-4-18), (3-5-4), (3-5-6), (3-5-10), (3-5-12), (3-5-16), (3-5-18), (3-6-4), (3-6-6), (3-6-10), (3-6-12), (3-6-16), (3-6-18), (3-7-4), (3-7-6), (3-7-10), (3-7-12), (3-7-16), (3-7-18), (3-8-4), (3-8-6), (3-8-10), (3-8-12), (3-8-16), or (3-8-18), and more preferably a compound represented by formula (3-1-6), (3-1-18), (3-2-6), (3-2-18), (3-3-6), (3-3-18), (3-4-6), (3-4-18), (3-5-6), (3-5-18), (3-6-6), (3-6-18), (3-7-6), (3-7-18), (3-8-6), or (3-8-18).

According to the production method of the present invention, by-products represented by formula (P), formula (Q), and formula (R) can be present in the reaction mixture, in addition to the target amidate compound (3).

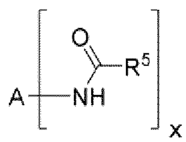

(P)

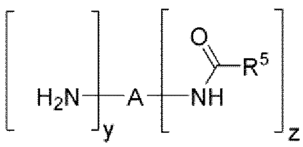

(Q)

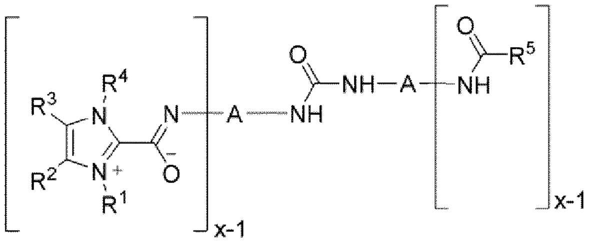

(R)

wherein $R^1$ to $R^5$, x, y, z, and A are as defined above.

The by-products represented by formula (P), formula (Q), and formula (R) may be separated to isolate the amidate compound (3) for use as a blocking agent dissociation catalyst for blocked isocyanates. Alternatively, a mixture comprising at least one by-product represented by formula (P), formula (Q), or formula (R), together with the amidate compound (3) may be used as a blocking agent dissociation catalyst for blocked isocyanates of the present invention. Additionally, a mixture comprising at least one by-product represented by Formula (P), formula (Q), or Formula (R), together with the amidate compound (3) can be mixed with a blocked isocyanate, and a compound having an isocyanate-reactive group to thus obtain a thermosetting resin composition of the present invention. Of the by-products represented by formula (P), formula (Q), and formula (R), the by-product represented by formula (R) has an amidate group as does the amidate compound (3), and is thus believed to function as a blocking agent dissociation catalyst for blocked isocyanates as does the amidate compound (3).

The mixtures comprising at least one by-product represented by formula (P), formula (Q), or formula (R), together with the amidate compound (3) are encompassed by the amidate compound (3) of the present invention.

Blocking Agent Dissociation Catalyst for Blocked Isocyanates

The amidate compound (3) can be used as a blocking agent dissociation catalyst for blocked isocyanates (hereinafter referred to as "the blocking agent dissociation catalyst"). The blocking agent dissociation catalyst is a catalyst that is capable of dissociating a blocking agent that blocks the isocyanate group of a blocked isocyanate and suppresses the reaction, and promoting the reaction between the regenerating isocyanate group and the coexisting isocyanate-reactive group.

When the amidate compound (3) is used as a blocking agent dissociation catalyst for blocked isocyanates, $R^1$ and $R^4$ in formula (3) are the same or different, and are each a $C_1$-$C_{20}$ hydrocarbon group optionally substituted with one or more heteroatoms, preferably, for example, a $C_1$-$C_{12}$ hydrocarbon group optionally substituted with one or more heteroatoms, and particularly preferably a $C_1$-$C_8$ hydrocarbon group optionally substituted with one or more heteroatoms. The hydrocarbon group is preferably an aliphatic hydrocarbon group, and more preferably an alkyl group. Examples of the $C_1$-$C_{20}$ hydrocarbon group optionally substituted with one or more heteroatoms include a methyl group, an ethyl group, a propyl group, an isopropyl group, a butyl group, a sec-butyl group, a tert-butyl group, a pentyl group, a hexyl group, a heptyl group, an octyl group, a 1,1,3,3-tetramethylbutyl group, a 1-ethylpentyl group, a 2-ethylhexyl group, a decyl group, a dodecyl group, a tetradecyl group, a hexadecyl group, an octadecyl group, an allyl group, a benzyl group, a cyclohexyl group, an adamantyl group, a phenyl group, a 2,6-diisopropylphenyl group, a 2,4,6-trimethylphenyl group, a 2-methoxyethyl group, a 2-ethoxyethyl group, 2-(dimethylamino)ethyl group, and the like. Preferred are a methyl group, an ethyl group, a propyl group, an isopropyl group, a butyl group, an octyl group, a dodecyl group, a cyclopentyl group, a cyclohexyl group, a 2-ethylhexyl group, a benzyl group, a phenyl group, and 2,4,6-trimethylphenyl group; and particularly preferred are a methyl group, an ethyl group, a butyl group, an octyl group, a 2-ethylhexyl group, and a benzyl group.

Examples of heteroatoms in $R^1$ and $R^4$ include nitrogen, oxygen, sulfur, and the like. When the hydrocarbon group is substituted with a heteroatom, such as oxygen, nitrogen, or sulfur, the hydrocarbon group has a group, such as -O-, -N<, -S-, or $-SO_2-$, and the hydrocarbon chain is interrupted by such a group. When the hydrocarbon group is substituted with a heteroatom, such as oxygen, nitrogen, or sulfur, it is preferred that the hydrocarbon group is substituted with oxygen and that the hydrocarbon chain is interrupted by an -O- group.

Next, the blocking agent dissociation catalyst comprising the amidate compound (3) is explained.

The blocking agent dissociation catalysts can be used singly or as a mixture of two or more. Further, a solvent or the like can be mixed and used, if necessary.

The solvent is not particularly limited. Examples include hydrocarbon solvents, such as benzene, toluene, xylene, cyclohexane, mineral spirit, and naphtha; ketone solvents, such as acetone, methyl ethyl ketone, and methyl isobutyl ketone; ester solvents, such as ethyl acetate, butyl acetate, and cellosolve acetate; alcohol solvents, such as methanol, ethanol, 2-propanol, butanol, 2-methoxyethanol, 2-ethoxyethanol, and 2-butoxyethanol; polyol solvents, such as ethylene glycol, propylene glycol, diethylene glycol, polyethylene glycol, and glycerol; water; and the like. These solvents may be used singly or in a combination of two or more.

The blocking agent dissociation catalyst of the present invention is a catalyst that promotes curing of a mixture of the blocked isocyanate and a compound having an isocyanate-reactive group.

The blocking agent dissociation catalyst of the present invention can sufficiently achieve the object of the present invention, as long as it contains the amidate compound (3) as an active ingredient. If necessary, the blocking agent dissociation catalyst of the present invention may contain a known blocking agent dissociation catalyst.

The blocking agent dissociation catalyst of the present invention can be preferably used, for example, as a catalyst in a method of dissociating the blocking agent of a blocked isocyanate. In this blocking agent dissociation method, a blocked isocyanate is heated in the presence of the blocking agent dissociation catalyst.

In the blocking agent dissociation method of the present invention, the amount of the blocking agent dissociation catalyst used is not particularly limited. The amount of the amidate compound (3) contained in the blocking agent dissociation catalyst is generally 0.01 to 15 wt%, preferably 0.05 to 10 wt%, and more preferably 0.1 to 5 wt%, relative to the solids content in the thermosetting resin composition described below.

In the present specification, "solids content" means the total mass of the components in a thermosetting resin composition, excluding the solvents described below. Thus, when a resin composition does not contain any solvent, the total mass of this composition is equal to its solids content.

The reaction temperature varies depending on the blocked isocyanate used, and is generally about 60 to 250°C, and preferably about 80 to 200°C. The reaction time is about 30 seconds to 5 hours, and preferably about 30 seconds to 2 hours.

Thermosetting Resin Composition

The thermosetting resin composition of the present invention comprises the amidate compound (3), a blocked isocyanate, and a compound having an isocyanate-reactive group.

Examples of blocked isocyanates include compounds obtained by reacting known polyisocyanates and a known blocking agent so that the isocyanate groups in the polyisocyanates are blocked with the blocking agent. The blocked isocyanates may be used singly or as a mixture of two or more.

In the present invention, the polyisocyanate is not particularly limited, as long as it is a compound having two or more isocyanate groups. Examples of known polyisocyanates include aliphatic polyisocyanates, alicyclic polyisocyanates, aromatic polyisocyanates, aromatic aliphatic polyisocyanates, modified isocyanates thereof, and the like. These polyisocyanates may be used singly or as a mixture of two or more.

Examples of aliphatic polyisocyanates include 1,4-tetramethylene diisocyanate, 1,6-hexamethylene diisocyanate, 2,2,4-trimethylhexamethylene diisocyanate, 2,4,4-trimethylhexamethylene diisocyanate, lysine diisocyanate, and the like.

Examples of alicyclic polyisocyanates include 1,3-bis(isocyanatomethyl)cyclohexane, 1,4-bis(isocyanatomethyl)cyclohexane, 3-isocyanatomethyl-3,3,5-trimethylcyclohexyl isocyanate (isophorone diisocyanate), bis-(4-isocyanatocyclohexyl)methane, norbornane diisocyanate, dimer acid diisocyanate, and the like.

Examples of aromatic polyisocyanates include 2,4'-diphenylmethane diisocyanate, 4,4'-diphenylmethane diisocyanate, crude diphenylmethane diisocyanate, 1,4-phenylene diisocyanate, 2,4-tolylene diisocyanate, 2,6-tolylene diisocyanate, 3,3'-dimethyl-4,4'-diisocyanatobiphenyl, 3,3'-dimethyl-4,4'-diisocyanatodiphenylmethane, 1,5-naphthylene diisocyanate, and the like.

Examples of aromatic aliphatic polyisocyanates include 1,3-xylylene diisocyanate, 1,4-xylylene diisocyanate, $\alpha,\alpha,\alpha',\alpha'$-tetramethylxylylene diisocyanate, and the like.

Examples of modified isocyanates include isocyanate-terminated compounds obtained by the reaction of the above polyisocyanate compounds with compounds having an active hydrogen group, and reaction products of the polyisocyanate compounds and/or the isocyanate-terminated compounds (e.g., adduct-type polyisocyanates, and modified isocyanates obtained by allophanatization reaction, carbodiimidization reaction, uretodionization reaction, isocyanuration reaction, uretoniminization reaction, biuretization reaction, or the like).

Examples of known blocking agents include alcohols, such as methanol, ethanol, propanol, isopropanol, butanol, sec-butanol, tert-butanol, 2-ethylhexanol, and butyl cellosolve; fluorinated alcohols, such as 2,2,2-trifluoroethanol and 1,1,1,3,3,3-hexafluoro-2-propanol; phenols, such as phenol, cresol, and 2-hydroxypyridine; amines, such as diisopropylamine; lactams, such as $\varepsilon$-caprolactam, $\delta$-valerolactam, and $\gamma$-butyrolactam; oximes, such as formaldehyde oxime, acetaldehyde oxime, acetone oxime, methyl ethyl ketoxime, and methyl isobutyl ketoxime; ketoenols, such as acetylacetone; pyrazoles, such as 1,2-pyrazole and 3,5-dimethylpyrazole; triazoles, such as triazole; and the like. Preferred are lactams, oximes, and pyrazoles; and particularly preferred are $\varepsilon$-caprolactam, methyl ethyl ketoxime, and 3,5-dimethylpyrazole.

Examples of the compound having an isocyanate-reactive group include compounds having two or more active hydrogen groups, such as polyols, polyamines, and alkanolamines. These compounds having an isocyanate-reactive group may be a mixture of two or more.

In the present invention, polyols are compounds having two or more hydroxyl groups. Examples of polyols include polyether polyols, polyester polyols, acrylic polyols, polyolefin polyols, fluorine polyols, polycarbonate polyols, polyurethane polyols, and the like. These polyols may be a mixture of two or more.

Examples of polyether polyols include active hydrogen compounds, such as aliphatic amine polyols, aromatic amine polyols, Mannich polyols, polyhydric alcohols, polyhydric phenols, and bisphenols; compounds obtained by adding alkylene oxides to these active hydrogen compounds; and the like. These polyether polyols may be a mixture of two or more.

Examples of aliphatic amine polyols include alkylenediamine-based polyols, alkanolamine-based polyols, and the like. These polyol compounds are polyfunctional polyol compounds having terminal hydroxyl groups obtained by the ring-opening addition of at least one cyclic ether, such as ethylene oxide or propylene oxide, using alkylenediamine or alkanolamine as an initiator. As the alkylenediamine, known compounds can be used without limitation. Specifically, $C_{2-8}$ alkylenediamines, such as ethylenediamine, propylenediamine, butylenediamine, hexamethylenediamine, and neopentyldiamine, are preferably used. These aliphatic amine polyols may be a mixture of two or more.

Aromatic amine polyols are polyfunctional polyether polyol compounds having terminal hydroxyl groups obtained by the ring-opening addition of at least one cyclic ether, such as ethylene oxide or propylene oxide, using an aromatic diamine as an initiator. As the initiator, a known aromatic diamine can be used without limitation. Specific examples include 2,4-toluenediamine, 2,6-toluenediamine, diethyltoluenediamine, 4,4'-diaminodiphenylmethane, p-phenylenediamine, o-phenylenediamine, naphthalenediamine, and the like. Among these, toluenediamine (2,4-toluenediamine, 2,6-toluenediamine, or a mixture thereof) is particularly preferably used. These aromatic amine polyols may be a mixture of two or more.

Mannich polyols are active hydrogen compounds obtained by the Mannich reaction of phenol and/or an alkyl-substituted derivative thereof, formaldehyde, and alkanolamine, or polyol compounds obtained by the ring-opening addition polymerization of the active hydrogen compounds with at least one of ethylene oxide and propylene oxide. These Mannich polyols may be a mixture of two or more.

Examples of polyhydric alcohols include dihydric alcohols (e.g., ethylene glycol, propylene glycol, 1,4-butanediol, 1,6-hexanediol, diethylene glycol, triethylene glycol, dipropylene glycol, and neopentyl glycol), trihydric or higher alcohols (e.g., glycerol, trimethylolpropane, pentaerythritol, methylglucoside, sorbitol, and sucrose), and the like. These polyhydric alcohols may be a mixture of two or more.

Examples of polyhydric phenols include pyrogallol, hydroquinone, and the like. These polyhydric phenols may be a mixture of two or more.

Examples of bisphenols include bisphenol A, bisphenol S, bisphenol F, low-condensates of phenols and formaldehyde, and the like. These bisphenols may be a mixture of two or more.

Examples of polyester polyols include polyester polyols obtained by the condensation reaction of a single or a mixture of dibasic acids selected from the group of carboxylic acids, such as succinic acid, adipic acid, sebacic acid, dimer acid, maleic anhydride, phthalic anhydride, isophthalic acid, and terephthalic acid, with a single or a mixture of polyhydric alcohols selected from the group of ethylene glycol, propylene glycol, diethylene glycol, neopentyl glycol, trimethylolpropane, glycerol, etc.; and polycaprolactones obtained by the ring-opening polymerization of $\varepsilon$-caprolactone using a polyhydric alcohol. These polyester polyols may be a mixture of two or more.

Acrylic polyols are compounds obtained by copolymerizing a single or a mixture of ethylenically unsaturated bond-containing monomers having a hydroxyl group with a single or a mixture of other ethylenically unsaturated bond-containing monomers copolymerizable therewith. Examples of the ethylenically unsaturated bond-containing monomer having a hydroxyl group include hydroxyethyl acrylate, hydroxypropyl acrylate, hydroxybutyl acrylate, hydroxyethyl methacrylate, hydroxypropyl methacrylate, hydroxybutyl methacrylate, and the like; and preferably hydroxyethyl acrylate and hydroxyethyl methacrylate. These acrylic polyols may be a mixture of two or more.

Examples of the other ethylenically unsaturated bond-containing monomers copolymerizable with the ethylenically unsaturated bond-containing monomer having a hydroxyl group include acrylates, such as methyl acrylate, ethyl acrylate, propyl acrylate, isopropyl acrylate, butyl acrylate, isobutyl acrylate, hexyl acrylate, cyclohexyl acrylate, 2-ethylhexyl acrylate, lauryl acrylate, benzyl acrylate, and phenyl acrylate; methacrylates, such as methyl methacrylate, ethyl methacrylate, propyl methacrylate, isopropyl methacrylate, n-butyl methacrylate, isobutyl methacrylate, hexyl methacrylate, cyclohexyl methacrylate, 2-ethylhexyl methacrylate, lauryl methacrylate, benzyl methacrylate, and phenyl methacrylate; unsaturated carboxylic acids, such as acrylic acid, methacrylic acid, maleic acid, and itaconic acid; unsaturated amides, such as acrylamide, methacrylamide, N,N-methylenebisacrylamide, diacetone acrylamide, diacetone methacrylamide, maleic acid amide, and maleimide; vinyl monomers, such as glycidyl methacrylate, styrene, vinyl toluene, vinyl acetate, acrylonitrile, and dibutyl fumarate; vinyl monomers having a hydrolyzable silyl group, such as vinyltrimethoxysilane, vinylmethyldimethoxysilane, and $\gamma$-(meth)acryloxypropyltrimethoxysilane; and the like.

Examples of polyolefin polyols include polybutadiene having two or more hydroxyl groups, hydrogenated polybutadiene, polyisoprene, hydrogenated polyisoprene, and the like. These polyolefin polyols may be a mixture of two or more.

Fluorine polyols are polyols containing fluorine in the molecule. Examples include copolymers of fluoroolefin, cyclovinyl ether, hydroxyalkyl vinyl ether, and vinyl monocarboxylate. These fluorine polyols may be a mixture of two or more.

Examples of polycarbonate polyols include those obtained by condensation polymerization of low-molecular-weight carbonate compounds, such as dialkyl carbonates (e.g., dimethyl carbonate), alkylene carbonates (e.g., ethylene carbonate), and diaryl carbonates (e.g., diphenyl carbonate), with low-molecular-weight polyols used in the polyester polyols described above. These polycarbonate polyols may be a mixture of two or more.

Polyurethane polyols can be obtained by a conventional method, for example, by reacting polyols and polyisocyanates. Examples of carboxyl group-free polyols include ethylene glycol and propylene glycol as low-molecular-weight polyols, and acrylic polyol, polyester polyol, and polyether polyol as high-molecular-weight polyols. These polyurethane polyols may be a mixture of two or more.

In the present invention, polyamines are compounds having two or more primary or secondary amino groups. Examples of polyamines include low-molecular-weight polyamines, high-molecular-weight polyamines, alkanolamines, and the like. These polyamines may be a mixture of two or more.

Examples of low-molecular-weight polyamines include aromatic amines, such as 4,4'-diphenylmethanediamine; araliphatic amines, such as 1,3- or 1,4-xylylenediamine and mixtures thereof; alicyclic amines, such as 3-aminomethyl-3,5,5-trimethylcyclohexylamine, 1,3-bis(aminomethyl)cyclohexane, and 1,4-cyclohexanediamine; aliphatic amines, such as ethylenediamine, 1,3-propanediamine, 1,4-butanediamine, 1,6-hexamethylenediamine, hydrazine, diethylenetriamine, triethylenetetramine, and tetraethylenepentamine; and the like. These low-molecular-weight polyamines may be a mixture of two or more.

Examples of high-molecular-weight polyamines include polyoxyalkylene diamine (weight average molecular weight: 400 to 4000), polyoxyalkylene triamine (weight average molecular weight: 400 to 5000), and the like. These high-molecular-weight polyamines may be a mixture of two or more.

Examples of alkanolamines include monoethanolamine, diethanolamine, N-(2-aminoethyl)ethanolamine, N-(2-hydroxypropyl)ethylenediamine, monopropanolamine, monoisopropanolamine, dipropanolamine, diisopropanolamine, ethylene glycol bis(3-aminopropyl)ether, neopentanolamine, methylethanolamine, and the like.

In the thermosetting resin composition of the present invention, the mixing ratio of the blocked isocyanate and the compound having an isocyanate-reactive group is determined by the required physical properties, and is not particularly limited. The mixing ratio is generally within the following range: [effective isocyanate groups (mol) in the blocked isocyanate]/[active hydrogen groups (mol) in the compound having an isocyanate-reactive group] = 0.2 to 3. The effective isocyanate groups in the blocked isocyanate refer to isocyanate groups that are regenerated when the blocking agent is dissociated from the blocked isocyanate.

In the thermosetting resin composition of the present invention, the amount of the blocking agent dissociation catalyst of the present invention used is not particularly limited. In the thermosetting resin composition, the amount of the blocking agent dissociation catalyst is generally such that the amount of the amidate compound (3) contained in the blocking agent dissociation catalyst is 0.01 to 15 wt%, preferably 0.05 to 10 wt%, and more preferably 0.1 to 5 wt%, relative to the solids content in the thermosetting resin composition.

In the thermosetting resin composition of the present invention, known catalysts for polyurethane production, additives, pigments, solvents, and the like that are commonly used in this technical field can be used, if necessary.

Known catalysts for polyurethane production are not particularly limited. Examples include tin compounds, such as dibutyltin dilaurate, dibutyltin di-2-ethylhexanate, dioctyltin dilaurate, dibutyltin diacetate, dibutyltin dioxide, dioctyltin dioxide, tin acetylacetonate, tin acetate, tin octylate, and tin laurate; bismuth compounds, such as bismuth octylate, bismuth naphthenate, and bismuth acetylacetonate; titanium compounds, such as tetra-n-butyl titanate, tetraisopropyl titanate, and titanium terephthalate; tertiary amine compounds, such as triethylamine, N,N,N',N'-tetramethylethylenediamine, N,N,N',N'-tetramethylpropylenediamine, N,N,N',N",N"-pentamethyldiethylenetriamine, N,N,N',N",N"-pentamethyldipropylenetriamine, N,N,N',N'-tetramethylguanidine, 1,3,5-tris(N,N-dimethylaminopropyl)hexahydro-S-triazine, 1,4-diazabicyclo[2.2.2]octane (DABCO), 1,8-diazabicyclo[5.4.0]undecene-7, triethylenediamine, N,N,N',N'-tetramethylhexamethylenediamine, N-methyl-N'-(2-dimethylaminoethyl)piperazine, N,N'-dimethylpiperazine, dimethylcyclohexylamine, N-methylmorpholine, N-ethylmorpholine, bis(2-dimethylaminoethyl)ether, 1-methylimidazole, 1,2-dimethylimidazole, 1-isobutyl-2-methylimidazole, and 1-dimethylaminopropylimidazole; and quaternary ammonium salt compounds, such as tetraalkylammonium halides (e.g., tetramethylammonium chloride), tetraalkylammonium hydroxides (e.g., tetramethylammonium hydroxide salts), tetraalkylammonium organic acid salts (e.g., tetramethylammonium-2-ethylhexanoate, 2-hydroxypropyl trimethylammonium formate, and 2-hydroxypropyl trimethylammonium-2-ethylhexanoate).

Additives are not particularly limited. Examples include hindered amine-based, benzotriazole-based, and benzophenone-based UV absorbers; perchlorate-based and hydroxylamine-based coloration inhibitors; hindered phenol-based, phosphorus-based, sulfur-based, and hydrazide-based antioxidants; tin-based, zinc-based, and amine-based urethanization catalysts; leveling agents, rheology control agents, pigment dispersants, and the like.

Pigments are not particularly limited. Examples include organic pigments, such as quinacridone-based, azo-based, and phthalocyanine-based pigments; inorganic pigments, such as titanium oxide, barium sulfate, calcium carbonate, and silica; and other pigments, such as carbon-based pigments, metal foil pigments, and rust-preventive pigments.

Solvents are not particularly limited. Examples include hydrocarbons, such as benzene, toluene, xylene, cyclohexane, mineral spirit, and naphtha; ketones, such as acetone, methyl ethyl ketone, and methyl isobutyl ketone; esters, such as ethyl acetate, butyl acetate, and cellosolve acetate; alcohols, such as methanol, ethanol, 2-propanol, butanol, 2-methoxyethanol, 2-ethoxyethanol, and 2-butoxyethanol; polyhydric alcohols, such as ethylene glycol, propylene glycol, diethylene glycol, polyethylene glycol, and glycerol; water; and the like. These solvents may be used singly or in a combination of two or more.

When storage at high temperatures is assumed, the thermosetting resin composition of the present invention may be divided into a blocked isocyanate and a compound having an isocyanate-reactive group to form two-part thermosetting compositions, and when used, the two-part thermosetting resin compositions may be mixed to be used as the thermosetting composition of the present invention. In such a case, the blocking agent dissociation catalyst can be added and used when the two-part thermosetting compositions are mixed, or the compound having an isocyanate-reactive group and the blocking agent dissociation catalyst can be mixed in advance.

The thermosetting resin composition of the present invention can be used as paints for, for example, automobiles, buildings, steel furniture and other metal products, musical instruments and other wood products, construction machines and other mechanical vehicles, sashes and other building materials, and office machines and other electrical appliances; coating materials, inks, adhesives, or pressure-sensitive adhesives for, for example, artificial leather and rubber rolls; sealants for, for example, electronic components; sealing materials for, for example, automobiles and buildings; molding materials for, for example, 3D printers; and the like.

Next, the method for curing the thermosetting resin composition of the present invention is explained.

In the method of the present invention, a mixture of a blocked isocyanate and a compound having an isocyanate-reactive group is heated in the presence of the blocking agent dissociation catalyst described above.

The reaction temperature varies depending on the blocked isocyanate used, but is generally about 60 to 250°C, and preferably about 80 to 200°C. The reaction time is about 30 seconds to 5 hours, and preferably about 1 minute to 60 minutes.

The cured product of the present invention can be produced through the method for curing the thermosetting resin composition of the present invention described above.

## Examples

The present invention is described in more detail with reference to Production Examples and Examples. However, the present invention is not limited to these Examples.

(I) $^1$H-NMR Analysis Conditions

Device: AV400 produced by Bruker Corporation
Frequency: 400 MHz

(II) Liquid Chromatography-Mass Spectrometry (hereinafter referred to as "LC-MS") Conditions

LC system: UltiMate 3000 produced by Thermo Fisher Scientific K.K.
Column: SUMIPAX CDS Z-CLUE (length: 50 mm, inner diameter: 3.0 mm, particle diameter: 2 μm), produced by Sumika Chemical
Analysis Service, Ltd.
Column temperature: 35°C
Detection method: Photodiode array (PDA) detector, 240 nm
Flow rate: 0.5 mL/min
Mobile phase: A = 10 mM aqueous ammonium formate solution, B = methanol
Gradient: See Table 1 below
Sample: sample (10 mg)/methanol (20 mL)
Sample injection volume: 1 μL
MS device: Exactive
Ionization: ESI+
Scanning range: m/z 50 to 1000

Table 1

| Time (min) | A (vol%) | B (vol%) |
|---|---|---|
| 0 | 70 | 30 |
| 10 | 20 | 80 |
| 25 | 5 | 95 |
| 27 | 5 | 95 |
| 29 | 70 | 30 |
| 29.5 | 70 | 30 |

(III) Curing Temperature Measurement Conditions

Device: Madoka automatic curing time measuring device produced by Cyber Co., Ltd.
Stirring rod: Model number 3JC-5060W
Stirring rate: rotation 100 rpm, revolutions 25 rpm

(IV) Measurement of NCO Group Content (%) in Polymeric MDI

The NCO group content (%) as used here refers to the amount of isocyanate groups present in a polyisocyanate expressed as a mass fraction. The NCO group content was measured and calculated according to the following method.

1.6341 g of polymeric MDI (Sumidur 44V20, produced by Sumika Covestro Urethane Co., Ltd.) was placed in a 200-mL conical flask, and 50 mL of a toluene solution of 0.2 mol/L dibutylamine was added thereto to dissolve the polymeric MDI. A small amount of bromocresol green was then added to the polymeric MDI solution, and a 0.5 mol/L ethanolic hydrochloric acid solution was added thereto dropwise with a burette. A blank test was also conducted in the same manner, except that polymeric MDI was not used. The amount of the ethanolic hydrochloric acid solution required for the solution in the flask to turn from blue to yellow was 50.17 mL in the blank test and 25.24 mL in the system that used polymeric MDI.

According to the following formula, the NCO group content in the polymeric MDI was calculated to be 32.0%.

NCO group content (%) = [{(titration volume of the ethanolic hydrochloric acid solution in the blank test (mL) - titration volume of the ethanolic hydrochloric acid solution in the sample (mL)} × concentration of the ethanolic hydrochloric acid solution (mol/mL)]/weight of polymeric MDI (g) × 4.202 = [{(50.17 (mL) - 25.24 (mL)} × 0.5 (mol/mL)]/1.6341 (g) × 4.202

(V) Calculation of Effective NCO Group Content (%)

The effective NCO group content (%) as used here is to quantify the amount of blocked isocyanate groups that can be involved in the crosslinking reaction and that are present in a blocked isocyanate after a blocking reaction. The effective NCO group content is expressed as a mass (%) of isocyanate groups and calculated according to the following formula:

Effective NCO group content (%) = {(solids content in blocked isocyanate (mass (%))) × (mass of polyisocyanate used in the reaction × NCO group content in precursor polyisocyanate (%))}/(mass of resin of blocked isocyanate after blocking reaction). When a solvent etc. was used for dilution, the values of those in the diluted state were used.

(VI) Calculation of Solids Content

About 1.5 g of a sample was heated at 110°C for 3 hours, and the solids content (%) in the sample was calculated from the mass before and after heating.

(VII) Formulation of Thermosetting Resin Composition

A blocked isocyanate, a polyol, and an amidate compound were added such that effective NCO group (mol):hydroxyl group (mol):amidate group (mol) = 1.00:0.95:0.05, and methyl isobutyl ketone was added such that the total solvent amount is 1.0 times by weight relative to the solids content in the blocked isocyanate. The effective NCO group (mol) and hydroxyl group (mol) were calculated according to the following formula.

Effective NCO group (mol) = amount of the blocked isocyanate used (g)/effective NCO group content (%) in the blocked isocyanate/4.202

Hydroxyl group (mol) = amount of the polyol used (g) × hydroxyl group value of the polyol (mgKOH/g)/56.1

Amidate Group

In the present specification, the skeleton represented by the following formula (A) is referred to as an "amidate group."

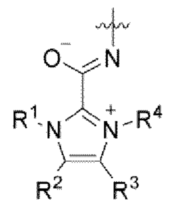

(A)

wherein $R^1$ to $R^4$ are as defined above.

In the Examples, the amidate group concentration was calculated according to the following method.

An internal standard substance (P mmol), such as tetralin or dimethyl sulfone, was added to an amidate compound (Q g), dissolved in any deuterated solvent, and analyzed by $^1$H-NMR. The integrated intensity (S) of the peaks corresponding to R number of hydrogen atoms of $R^1$ and $R^4$ of the amidate group (A) bonded to the carbon atoms adjacent to the nitrogen atoms of the imidazolium skeleton and the integrated intensity (U) of the peaks corresponding to T number of hydrogen atoms bonded to any group in the internal standard substance were determined to calculate the amidate group concentration according to the following formula.

$$\text{Amidate group concentration (mmol/g)} = P \times S \times T/(R \times U \times Q)$$

In the Examples, wt% indicates mass%.

Production Example 1: Synthesis of [D2EHI][OAc]

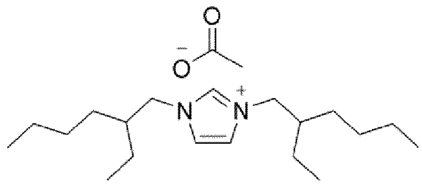

52.3 g (0.87 mol) of acetic acid, 42.1 g (0.56 mol) of 41 wt% formalin aqueous solution, and 82.8 g (0.58 mol) of 41 wt% glyoxal aqueous solution were placed in a 500-mL four-necked reactor purged with nitrogen, and the mixture was heated to 50°C. Subsequently, 150.1 g (1.16 mol) of 2-ethylhexylamine was added dropwise to the reactor over 2 hours, and the mixture was stirred for another 2 hours and 30 minutes. Then, 3.4 g (0.05 mol) of 41 wt% formalin aqueous solution and 6.7 g (0.05 mol) of 41 wt% glyoxal aqueous solution were added to the reaction solution, followed by stirring for another 30 minutes. The obtained reaction solution was concentrated under reduced pressure to give 225.8 g of a dark-brown viscous liquid. The results of $^1$H-NMR analysis of the obtained dark-brown viscous liquid with tetralin added as an internal standard substance revealed that the obtained dark-brown viscous liquid contained 198.4 g (0.56 mol, yield: 96.9%) of [D2EHI][OAc] represented by the above formula, and 25.2 g (0.42 mol) of acetic acid. The $^1$H-NMR analysis results of [D2EHI][OAc] are shown below.

$^1$H-NMR(DMSO-$d_6$)$\delta$(ppm)=9.35 (s, 1H), 7.82 (s, 2H), 4.15 (d, J=7.2 Hz, 4H), 1.84 (m, 2H), 1.71 (s, 3H), 1.25 (m, 16H), 0.87 (t, J=7.2 Hz, 12H)

Production Example 2: Synthesis of [D2EHI][2EHA]

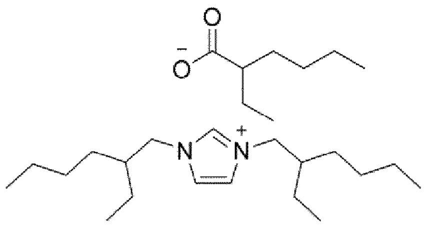

25.1 g (0.17 mol) of 2-ethylhexanoic acid, 8.6 g (0.12 mol) of 41 wt% formalin aqueous solution, and 16.8 g (0.12 mol) of 41 wt% glyoxal aqueous solution were placed in a 200-mL three-necked reactor purged with nitrogen, and the mixture was heated to 80°C. Subsequently, 30.0 g (0.23 mol) of 2-ethylhexylamine was added dropwise at 80°C to the reactor over 2 hours, and the mixture was stirred for 2 hours. Then, 0.9 g (0.01 mol) of 41 wt% formalin aqueous solution and 1.7 g (0.01 mol) of 41 wt% glyoxal aqueous solution were added to the reaction solution, followed by stirring for another 1 hour and 30 minutes. The obtained reaction solution was concentrated under reduced pressure to give 59.6 g of a dark-brown viscous liquid. The results of $^1$H-NMR analysis of the obtained dark-brown viscous liquid with tetralin added as an internal standard substance revealed that the obtained dark-brown viscous liquid contained 35.4 g (0.08 mol, yield: 72.0%) of [D2EHI][2EHA] represented by the above formula and 12.1 g (0.08 mol) of 2-ethylhexanoic acid. The $^1$H-NMR analysis results of [D2EHI][2EHA] are shown below.

$^1$H-NMR($CDCl_3$)$\delta$(ppm)=10.91-10.84 (m, 1H), 7.07 (s, 2H), 4.33-4.21 (m, 4H), 2.23-2.15 (m, 2H), 1.83-1.77 (m, 2H), 1.64-1.54 (m, 4H), 1.48-1.28 (m, 20H), 0.87 (t, J=7.2 Hz, 12H)

Production Example 3: Synthesis of [D2EHI][OAc]

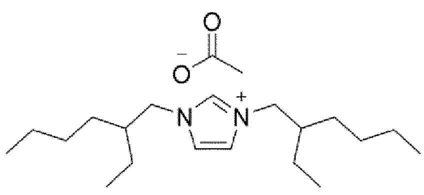

40.0 g (pure content: 99.5 mmol) of [D2EHI][OAc] obtained in Production Example 1 and 39.9 g (443 mmol) of

dimethyl carbonate were placed in a 2-L four-necked reactor purged with nitrogen, and the mixture was stirred under reflux for 5 hours. The obtained reaction solution was concentrated under reduced pressure to give 34.1 g of a dark-brown viscous liquid. The results of $^1$H-NMR analysis of the obtained dark-brown viscous liquid with dimethyl sulfone added as an internal standard substance revealed that the obtained dark-brown viscous liquid contained 31.7 g (83.8 mmol, yield: 84.2%) of [D2EHI][OAc] represented by the above formula, and that the excess acetic acid was eliminated.

Production Example 4: Synthesis of [D2EHI][2EHA]

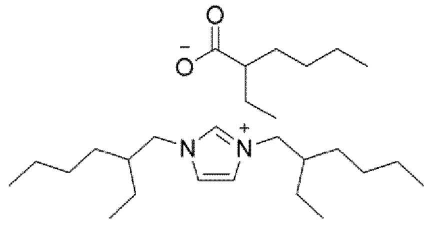

16.2 g (pure content: 22.7 mmol) of [D2EHI][2EHA] obtained in Production Example 2 and 16.2 g (180 mmol) of dimethyl carbonate were placed in a 100-mL three-necked reactor purged with nitrogen, and the mixture was stirred at 90°C for 4 hours. The obtained reaction solution was concentrated under reduced pressure to give 12.4 g of a dark-brown viscous liquid. The results of $^1$H-NMR analysis of the obtained dark-brown viscous liquid with tetralin added as an internal standard substance revealed that the obtained dark-brown viscous liquid contained 9.7 g (22.1 mmol, yield: 97.3%) of [D2EHI] [2EHA] represented by the above formula, and that the excess 2-ethylhexanoic acid was eliminated.

Production Example 5: Synthesis of MEKO-blocked HDI biuret

60.0 g (NCO group: 326 mmol) of HDI biuret (Desmodur N3200A, NCO group content: 22.8%, produced by Sumika Covestro Urethane Co., Ltd.) and 36.9 g of methyl isobutyl ketone were placed in a 200-mL three-necked reactor purged with nitrogen and heated to 65°C. After heating, 0.6 g of triethylamine was added to the reactor. Thereafter, 27.0 g (333 mmol) of methyl ethyl ketoxime and 22.9 g of methyl isobutyl ketone were added dropwise to the reactor, and the mixture was stirred for 2 hours. The obtained reaction solution was concentrated under reduced pressure, and 17.4 g of methyl isobutyl ketone was added to give 119.0 g of a MEKO-blocked HDI biuret. The obtained MEKO-blocked HDI biuret had a solids content of 74.7 % and an effective NCO group content of 11.6 %.

Example 1: Synthesis of D2EHIm_TDI_Me

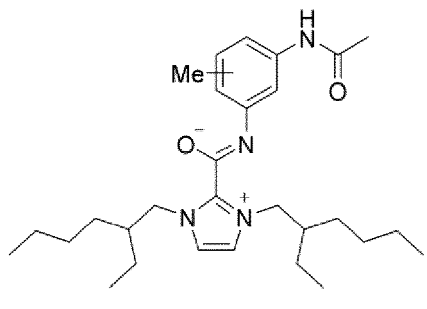

30.0 g of toluene was placed in a 200-mL three-necked reactor purged with nitrogen and heated under reflux. Subsequently, a mixed solution of 30.0 g (pure content: 79.3 mmol) of [D2EHI] [OAc] obtained in Production Example 3 and 30.0 g of toluene, and a mixed solution of 15.7 g (89.9 mmol) of tolylene diisocyanate (a mixture of about 80% of 2,4-tolylene diisocyanate and about 20% of 2,6-tolylene diisocyanate, produced by Tokyo Chemical Industry Co., Ltd.) and 30.0 g of toluene were added dropwise to the reactor over 2 hours and stirred for 2 hours. After stirring, the obtained reaction mixture was concentrated to give 38.1 g of a mixture containing the compound (D2EHIm_TDI_Me) represented by the above formula as a dark-brown viscous liquid. Further, broadening and multiplet splitting were observed in the

$^1$H-NMR peaks, which suggested that reaction products of [D2EHI] [OAc] and a modified isocyanate in which some of the isocyanate groups of the tolylene diisocyanate used as a starting material were oligomerized were also produced as by-products. The results of $^1$H-NMR analysis of the obtained dark-brown viscous liquid with dimethyl sulfone added as an internal standard substance revealed that, assuming that the peak (4.53-4.36 ppm) corresponding to the four hydrogen atoms of the methylene groups adjacent to the nitrogen atoms of the imidazolium group were all derived from D2EHIm_TDI_Me represented by the above formula, the dark-brown viscous liquid contained 30.3 g (62.5 mmol, yield: 78.7 %) of D2EHIm_TDI_Me represented by the above formula, and had an amidate group concentration of 1.640 mmol/g. The results of $^1$H-NMR and mass spectrometry (LC-MS) of the target product (main product) obtained by ion chromatography, as well as the results of mass spectrometry (LC-MS) of the two by-products, are shown below.

Target Product (Main Product)

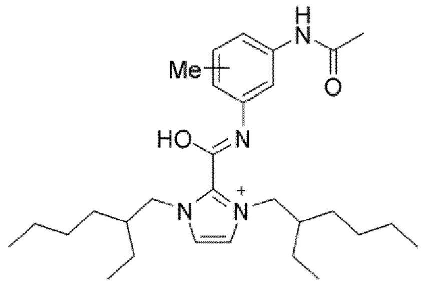

$^1$H-NMR($CDCl_3$)δ(ppm)=7.43-6.90 (m, 5H), 4.53-4.36 (m, 4H), 2.22-1.91 (m, 8H), 1.37-1.26 (m, 16H), 0.88-0.79 (m, 12H), LC-MS: calculated value of $C_{29}H_{47}N_4O_2^+$ = 483.3694, measured value (M+H$^+$) = 483.3668

By-products

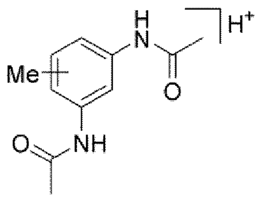

LC-MS: calculated value of $C_{11}H_{15}N_2O_2^+$ = 207.1128, measured value (M+H$^+$) = 207.1119

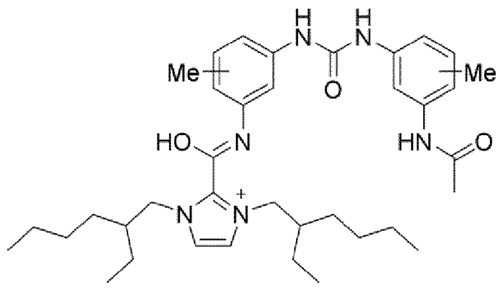

LC-MS: calculated value of $C_{37}H_{55}N_6O_3^+$ = 631.4330, measured value (M+H$^+$) = 631.4300

For the blocking agent dissociation catalyst of the present invention, only the target product may be isolated for use; however, mixtures comprising the target product and by-products can also sufficiently play the role of a blocking agent dissociation catalyst in thermosetting resin compositions.

Example 2: Synthesis of D2EHIm_TDI_2EH

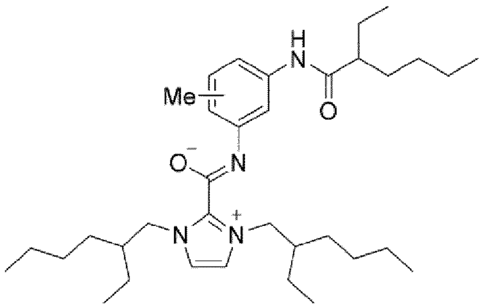

10.5 g of toluene was placed in a 100-mL three-necked reactor purged with nitrogen and heated under reflux. Subsequently, a mixed solution of 10.0 g (pure content: 17.8 mmol) of [D2EHI] [2EHA] obtained in Production Example 4 and 10.0 g of toluene, and a mixed solution of 3.1 g (17.9 mmol) of tolylene diisocyanate (a mixture of about 80 % of 2,4-tolylene diisocyanate and about 20% of 2,6-tolylene diisocyanate, produced by Tokyo Chemical Industry Co., Ltd.) and 10.1 g of toluene were added dropwise to the reactor over 2 hours and stirred for 1 hour. After stirring, the obtained reaction mixture was concentrated to give 9.1 g of a mixture containing the compound (D2EHIm_TDI_2EH) represented by the above formula as a dark-brown viscous liquid. The results of $^1$H-NMR analysis of the obtained dark-brown viscous liquid with tetralin added as an internal standard substance revealed that, assuming that the four hydrogen atoms of the methylene groups adjacent to the nitrogen atoms of the imidazolium group were all derived from D2EHIm_TDI_2EH represented by the above formula, the dark-brown viscous liquid contained 5.1 g (9.1 mmol, yield: 51.2%) of D2EHIm_TDI_2EH represented by the above formula, and had an amidate group concentration of 1.000 mmol/g. The results of $^1$H-NMR and mass spectrometry (LC-MS) of the target product (main product) obtained by ion chromatography, as well as the results of mass spectrometry (LC-MS) of two by-products, are shown below.

Target Product (Main Product)

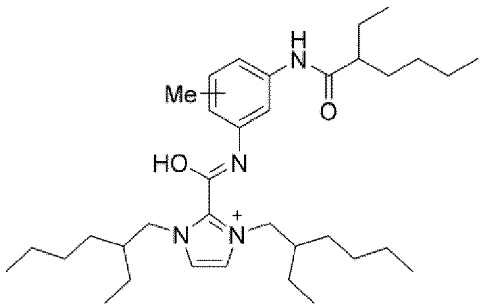

$^1$H-NMR($CDCl_3$)$\delta$(ppm)=7.27-6.86 (m, 5H), 4.54-4.47 (m, 4H), 2.24-2.15 (m, 3H), 1.94-1.86 (m, 1H), 1.78-1.62 (m, 2H), 1.39-1.19 (m, 24H), 1.01-0.81 (m, 18H)

LC-MS: calculated value of $C_{35}H_{59}N_4O_2^+$ = 567.4633, measured value ($M+H^+$) = 567.4598

By-products

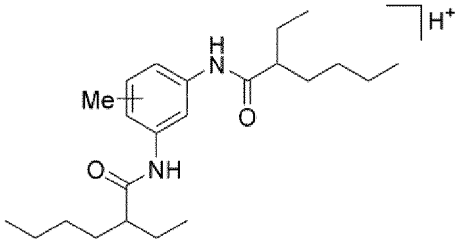

LC-MS: calculated value of $C_{23}H_{39}N_2O_2^+$ = 375.3006, measured value (M+H$^+$) = 375.2982

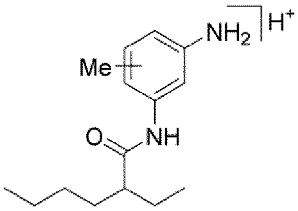

LC-MS: calculated value of $C_{15}H_{25}N_2O^+$ = 249.1961, measured value (M+H$^+$) = 249.1947

Example 3: Synthesis of D2EHIm_mMDI_Me

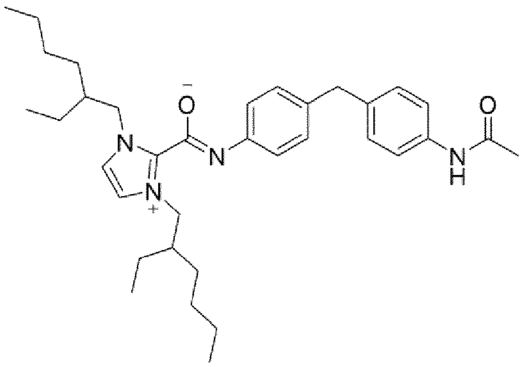

3.0 g of toluene was placed in a 30-mL three-necked reactor purged with nitrogen and heated under reflux. Subsequently, a mixed solution of 5.0 g (pure content: 13.2 mmol) of [D2EHI] [OAc] obtained in Production Example 3 and 5.9 g of toluene, and a mixed solution of 3.7 g (14.8 mmol) of 4,4'-diphenylmethane diisocyanate (produced by Tokyo Chemical Industry Co., Ltd.) and 5.0 g of toluene were added dropwise to the reactor over 2 hours and stirred for 1 hour. After stirring, the obtained reaction mixture was concentrated to give 8.3 g of a mixture containing the compound (D2EHIm_mMDI_Me) represented by the above formula as a brown viscous liquid. The results of $^1$H-NMR and mass spectrometry (LC-MS) of the target product (main product) obtained by ion chromatography, as well as the results of mass spectrometry (LC-MS) of three by-products, are shown below.

Target Product (Main Product)

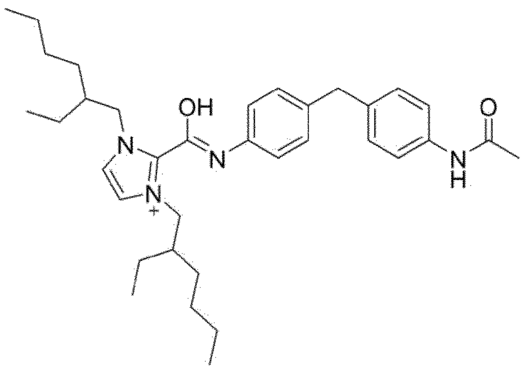

$^1$H-NMR($CDCl_3$)δ(ppm)=7.43-7.06 (m, 8H), 6.86 (s, 2H), 4.47 (m, 4H), 2.36 (s, 3H), 2.12 (s, 2H), 1.86 (m, 2H), 1.31 (m, 16H), 0.91 (m, 12H)

LC-MS: calculated value of $C_{35}H_{51}N_4O_2^+$ = 559.4007, measured value (M+H$^+$) = 559.3976

By-products

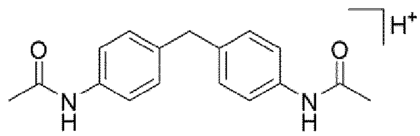

LC-MS: calculated value of $C_{17}H_{19}N_2O_2^+$ = 283.1441, measured value (M+H$^+$) = 283.1426

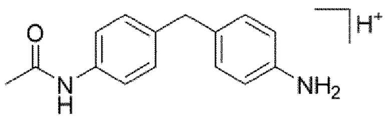

LC-MS: calculated value of $C_{15}H_{17}N_2O^+$ = 241.1335, measured value (M+H$^+$) = 241.1325

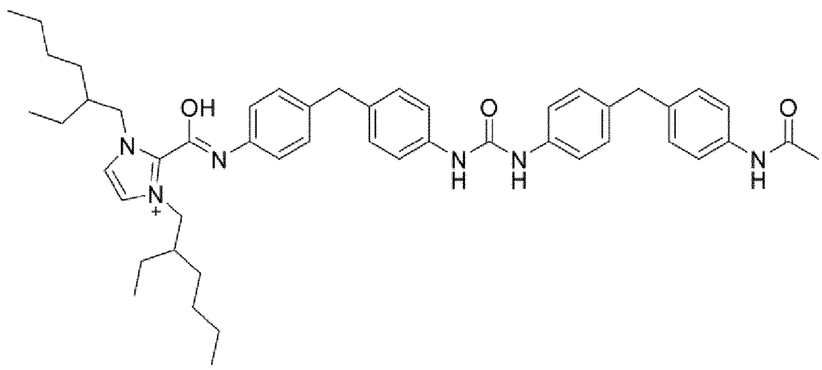

LC-MS: calculated value of $C_{49}H_{63}N_6O_3^+$ = 783.4956, measured value (M+H$^+$) = 783.4919

Example 4: Synthesis of D2EHIm_crMDI_Me

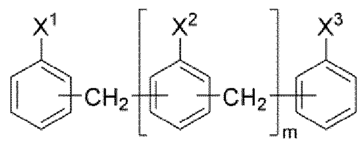

$X^1$, $X^2$, $X^3$ =

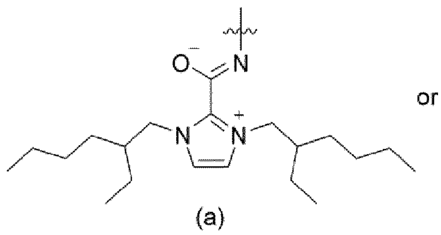

or

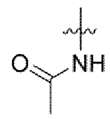

(a) (b)

In the formula, at least any one of $X^1$ to $X^3$ is substituted with a group represented by (a), and the rest is/are substituted with (b). Although the reaction mixture may contain a compound in which $X^1$ to $X^3$ are all substituted with (a), or a compound in which $X^1$ to $X^3$ are all substituted with (b), the main component of the reaction mixture is a compound substituted with at least one (a) and at least one (b). m is an integer of 0 to 4.

30.0 g of toluene was placed in a 180-mL three-necked reactor purged with nitrogen and heated under reflux. Subsequently, a mixed solution of 30.0 g (pure content: 0.075 mol) of [D2EHI][OAc] obtained in Production Example 1 and 30.0 g of toluene, and a mixed solution of 26.1 g (NCO group: 198.9 mmol) of polymeric MDI (Sumidur 44V20, NCO group content: 32.0%, produced by Sumika Covestro Urethane Co., Ltd.) and 24.0 g of toluene were added dropwise to the reactor over 2 hours and stirred for 1 hour. After stirring, the obtained reaction mixture was concentrated to dryness to give 48.6 g of a mixture containing the compound (D2EHIm_crMDI_Me) represented by the above formula as a brown solid. The results of $^1$H-NMR analysis of the obtained brown solid with tetralin added as an internal standard substance revealed that, in view of the four hydrogen atoms of the methylene groups adjacent to the nitrogen atoms of the imidazolium group, the brown solid had an amidate group concentration of 0.765 mmol/g. The $^1$H-NMR analysis results of the obtained mixture are shown below.

$^1$H-NMR($CDCl_3$)$\delta$(ppm)=7.41-6.88 (m), 4.46-4.36 (m)3.94-3.87 (m), 2.12 (s), 2.03-1.88 (m), 1.38-1.10 (m), 0.90-0.73 (m)

Example 5: Synthesis of D2EHIm_crMDI_2EH

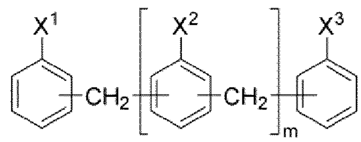

$X^1$, $X^2$, $X^3$ =

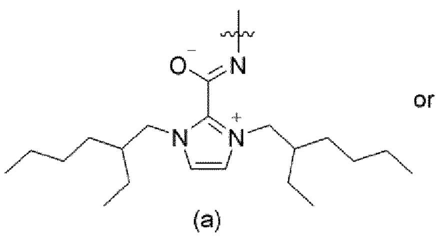

or

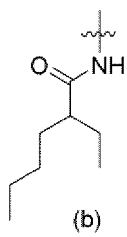

(a) (b)

In the formula, at least any one of $X^1$ to $X^3$ is substituted with a group represented by (a), and the rest is/are substituted with (b). Although the reaction mixture may contain a compound in which $X^1$ to $X^3$ are all substituted with (a), or a compound in which $X^1$ to $X^3$ are all substituted with (b), the main component of the reaction mixture is a compound

substituted with at least one (a) and at least one (b). m is an integer of 0 to 4. 30.0 g of toluene was placed in a 180-mL three-necked reactor purged with nitrogen and heated under reflux. Subsequently, a mixed solution of 30.0 g (pure content: 42.1 mmol) of [D2EHI] [2EHA] obtained in Production Example 2 and 30.0 g of toluene, and a mixed solution of 17.6 g (NCO group: 134.1 mol) of polymeric MDI (Sumidur 44V20, NCO group content: 32.0%, produced by Sumika Covestro Urethane Co., Ltd.) and 24.0 g of toluene were added dropwise to the reactor over 2 hours and stirred for 1 hour. After stirring, the obtained reaction mixture was concentrated to dryness to give 50.1 g of a mixture containing the compound (D2EHIm_crMDI_2EH) represented by the above formula as a brown solid. The results of $^{1}$H-NMR analysis of the obtained brown solid with tetralin added as an internal standard substance revealed that, in view of the four hydrogen atoms of the methylene groups adjacent to the nitrogen atoms of the imidazolium group, the brown solid had an amidate group concentration of 0.622 mmol/g. The $^{1}$H-NMR analysis results of the obtained mixture are shown below.

$^{1}$H-NMR($CDCl_3$)$\delta$(ppm)=7.50-6.89 (m), 4.53-4.37 (m), 4.01-3.81 (m), 2.13-2.05 (m), 1.93-1.84 (m), 1.76-1.65 (m), 1.60-1.47 (m), 1.40-1.28 (m), 0.99-0.80 (m)

Evaluation Example 1

The MEKO (methyl ethyl ketone oxime)-blocked HDI biuret obtained in Production Example 5, a polyester polyol (P-510, produced by Kuraray Co., Ltd.), and D2EHIm_TDI_Me obtained in Example 1 were added such that the formulation of the thermosetting resin composition satisfied that effective NCO group (mol):hydroxyl group (mol):amidate group (mol) = 1.00:0.95:0.05. Then, methyl isobutyl ketone was added such that the total solvent amount was 1.0 times by weight relative to the solids content in the blocked isocyanate, and the mixture was stirred for 30 minutes, thus preparing a thermosetting resin composition.

About 0.6 mL of the prepared thermosetting resin composition was collected and added to the hot plate of the automatic curing time measuring device that had been heated beforehand to a predetermined temperature, and stirring was performed. During this procedure, the curing time at each temperature was measured, taking the time when the stirring torque exceeded 20% (0.86 mN•m) as the curing time. Table 2 shows the results.

Evaluation Examples 2 to 5

Thermosetting resin compositions were prepared in the same manner as in Evaluation Example 1, except that D2EHIm_TDI_Me was changed to the amidate compounds shown in Table 2, and the curing time was measured. Table 2 shows the results.

Comparative Example 1

A thermosetting resin composition was prepared in the same manner as in Evaluation Example 1, except that D2EHIm_TDI_Me was changed to dibutyltin dilaurate (below, "DBTDL") such that the formulation of the thermosetting resin composition satisfied that effective NCO group (mol):hydroxyl group (mol):DBTDL (mol) = 1.00:0.95:0.05, and the curing time was measured. Table 2 shows the results.

Table 2

| | | Evaluation Example 1 | Evaluation Example 2 | Evaluation Example 3 | Evaluation Example 4 | Comparative Example 1 |
|---|---|---|---|---|---|---|
| Preparation of thermosetting resin com position | Blocked isocyanate(1) | 5.00 g | 5.00 g | 5.00 g | 5.00 g | 5.00 g |
| | Polyol(2) | 3.20 g | 3.20 g | 3.20 g | 3.20 g | 3.20 g |
| | Catalyst | D2EHIm_ TDI_Me obtained in Example 1 | D2EHIm_TDI_ 2EH obtained in Example 2 | D2EHIm_ crMDI_Me obtained in Example 4 | D2EHIm_ crMDI_2EH obtained in Example 5 | Dibutyltin dilaurate |
| | | 0.42 g | 0.69 g | 0.90 g | 1.11 g | 0.44 g |
| | Methyl isobutyl ketone | 2.47 g | 2.47 g | 2.47 g | 2.47 g | 2.47 g |

(continued)

| | | Evaluation Example 1 | Evaluation Example 2 | Evaluation Example 3 | Evaluation Example 4 | Comparative Example 1 |
|---|---|---|---|---|---|---|
| Curing time(3) | 140°C | - | - | - | - | 13 min |
| | 130°C | 7min | 7 min | 8 min | 7min | 29 min |
| | 120°C | 11 min | 11 min | 12 min | 13 min | - |
| | 110°C | 17 min | 18 min | 21 min | 26 min | - |
| | 100°C | 27 min | 30 min | 30 min | 46 min | - |
| (1) MEKO-blocked HDI biuret obtained in Production Example 5<br>(2) Polyester polyol P-510, produced by Kuraray Co., Ltd.<br>(3) In the table, "-" indicates no data. | | | | | | |

## Claims

**1.** A method for producing an amidate compound, the method comprising

reacting an imidazolium carboxylic acid salt represented by the following formula (1):

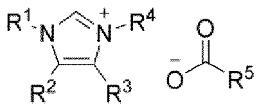
(1)

wherein
$R^1$ and $R^4$ are the same or different, and are each a $C_1$-$C_{20}$ hydrocarbon group optionally substituted with one or more heteroatoms,
$R^2$ and $R^3$ are the same or different, and are each a hydrogen atom or a $C_1$-$C_{20}$ hydrocarbon group optionally substituted with one or more heteroatoms, or $R^2$ and $R^3$, together with the carbon atoms to which they are attached, may form a ring structure, and
$R^5$ is a hydrogen atom or a $C_1$-$C_{20}$ hydrocarbon group optionally substituted with one or more heteroatoms, with
a polyisocyanate compound represented by the following formula (2) :

$$A\left[NCO\right]_x \quad (2)$$

wherein
A is a residue obtained by removing isocyanate groups from at least one polyisocyanate selected from the group consisting of aliphatic polyisocyanates, alicyclic polyisocyanates, aromatic polyisocyanates, and aromatic aliphatic polyisocyanates, or a residue obtained by removing isocyanate groups from a modified isocyanate formed from at least one member selected from the group consisting of aliphatic polyisocyanates, alicyclic polyisocyanates, aromatic polyisocyanates, and aromatic aliphatic polyisocyanates, and
x is an integer of 2 or more and 20 or less,
wherein the amidate compound is represented by the following formula (3):

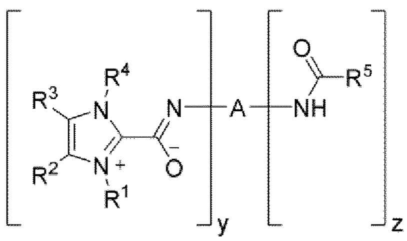
(3)

wherein y and z are each an integer of 1 or more and 19 or less, and the sum of y and z is 2 or more and 20 or less, and
A, $R^1$, $R^2$, $R^3$, $R^4$, and $R^5$ are as defined above.

**2.** The method for producing an amidate compound according to claim 1, wherein the polyisocyanate compound represented by formula (2) is an aromatic polyisocyanate.

**3.** The method for producing an amidate compound according to claim 1, wherein the polyisocyanate compound represented by formula (2) is a dimeric or trimeric polyisocyanate formed from at least one member selected from the group consisting of 2,4-tolylene diisocyanate, 2,6-tolylene diisocyanate, 4,4'-diphenylmethane diisocyanate, and polymethylene polyphenyl polyisocyanate.

**4.** The method for producing an amidate compound according to claim 1, wherein the polyisocyanate compound represented by formula (2) is at least one polyisocyanate selected from the group consisting of 2,4-tolylene diisocyanate, 2,6-tolylene diisocyanate, 4,4'-diphenylmethane diisocyanate, and polymethylene polyphenyl polyisocyanate.

**5.** The method for producing an amidate compound according to any one of claims 1 to 4, wherein $R^2$ and $R^3$ are each a hydrogen atom.

**6.** An amidate compound represented by formula (3):

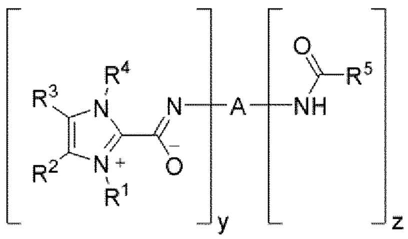

(3)

wherein

y and z are each an integer of 1 or more and 19 or less, and the sum of y and z is 2 or more and 20 or less, and
$R^1$ and $R^4$ are the same or different, and are each a $C_1$-$C_{20}$ hydrocarbon group optionally substituted with one or more heteroatoms,
$R^2$ and $R^3$ are the same or different, and are each a hydrogen atom or a $C_1$-$C_{20}$ hydrocarbon group optionally substituted with one or more heteroatoms, or $R^2$ and $R^3$, together with the carbon atoms to which they are attached, may form a ring structure, and
$R^5$ is a hydrogen atom or a $C_1$-$C_{20}$ hydrocarbon group optionally substituted with one or more heteroatoms.

**7.** The amidate compound according to claim 6, wherein $R^2$ and $R^3$ are each a hydrogen atom.

**8.** The amidate compound according to claim 6, wherein $R^1$ and $R^4$ are each a $C_1$-$C_{20}$ alkyl group optionally substituted with one or more heteroatoms.

**9.** A blocking agent dissociation catalyst for blocked isocyanates, comprising the amidate compound of any one of claims 6 to 8.

**10.** A thermosetting resin composition comprising the amidate compound of any one of claims 6 to 8, a blocked isocyanate, and a compound having an isocyanate-reactive group.

**11.** A cured product obtained by curing the thermosetting resin composition of claim 10.

**12.** A method for producing a cured product, the method comprising the step of heating and curing the thermosetting resin composition of claim 10.

**INTERNATIONAL SEARCH REPORT**

International application No.
PCT/JP2021/013184

A. CLASSIFICATION OF SUBJECT MATTER
Int. Cl. C07D233/90(2006.01)i, C08G18/20(2006.01)i, C08G18/76(2006.01)i, C08G18/80(2006.01)i
FI: C07D233/90 C CSP, C08G18/20, C08G18/76, C08G18/80
According to International Patent Classification (IPC) or to both national classification and IPC

B. FIELDS SEARCHED
Minimum documentation searched (classification system followed by classification symbols)
Int. Cl. C07D233/90, C08G18/20, C08G18/76, C08G18/80

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
CAplus/REGISTRY (STN); CASREACT (STN)

C. DOCUMENTS CONSIDERED TO BE RELEVANT

| Category* | Citation of document, with indication, where appropriate, of the relevant passages | Relevant to claim No. |
|---|---|---|
| X<br>A | COUTELIER, O. et al. N-Heterocyclic Carbene-catalyzed synthesis of Polyurethanes. Polymer Preprints. 2011, vol. 52, no. 2, pp. 290-291, p. 290, right column, Bipolar Ionic NHC-1-HD1 Additive, fig. 2 | 6-8<br>1-5, 9-12 |
| A | WO 2019/066029 A1 (KANSAI PAINT CO., LTD.) 04 April 2019, paragraph [0118]-[0122], production example 10 | 1-12 |
| P, A | WO 2020/067431 A1 (KOEI CHEMICAL COMPANY, LIMITED) 02 April 2020, claims, preparation example 6 | 1-12 |
| A | WO 2018/181753 A1 (KOEI CHEMICAL COMPANY, LIMITED) 04 October 2018, claims, examples | 1-12 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
10.05.2021

Date of mailing of the international search report
18.05.2021

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

**INTERNATIONAL SEARCH REPORT**

International application No.
PCT/JP2021/013184

C (Continuation). DOCUMENTS CONSIDERED TO BE RELEVANT

| Category* | Citation of document, with indication, where appropriate, of the relevant passages | Relevant to claim No. |
|---|---|---|
| A | WO 2019/065953 A1 (KOEI CHEMICAL COMPANY, LIMITED) 04 April 2019, claims, examples | 1-12 |

Form PCT/ISA/210 (continuation of second sheet) (January 2015)

**INTERNATIONAL SEARCH REPORT**
Information on patent family members

International application No.
PCT/JP2021/013184

| Patent Documents referred to in the Report | Publication Date | Patent Family | Publication Date |
|---|---|---|---|
| WO 2019/066029 A1 | 04.04.2019 | CN 111094471 A<br>paragraphs [0150]-[0154], [0257]-[0262] | |
| WO 2020/067431 A1 | 02.04.2020 | (Family: none) | |
| WO 2018/181753 A1 | 04.10.2018 | US 2020/0024237 A1<br>claims, examples<br>TW 201841894 A<br>KR 10-2019-0136022 A<br>EP 3604287 A1<br>CN 110475765 A | |
| WO 2019/065953 A1 | 04.04.2019 | US 2020/0216600 A1<br>claims, examples<br>TW 201920118 A<br>KR 10-2020-0060367 A<br>CN 111094375 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019065953 A1

### Non-patent literature cited in the description

- *Struct. Chem.,* 2013, vol. 24, 2059-2068